# EUROPEAN PATENT SPECIFICATION

(11) **EP 2 542 677 B1**
(45) Date of publication and mention of the grant of the patent: **01.07.2015**
(21) Application number: 11704847.0
(22) Date of filing: 16.02.2011
(51) Int. Cl.: C12N 15/113, A61K 31/7088, A61K 31/403

(54) **Molecular targets for healing or treating wounds**
Molekulare Targets zur Heilung oder Behandlung von Wunden
Cibles moléculaires pour la cicatrisation ou le traitement de plaies

(30) Priority: 05.03.2010 GB 201003652
(43) Date of publication of application: 09.01.2013
(73) Proprietor: University College Cardiff Consultants Limited, Cardiff South Glamorgan CF24 0DE (GB)
(72) Inventor: HARDING, Keith, Cardiff South Glamorgan CF23 9BJ (GB); JIANG, Wen Guo, Cardiff South Glamorgan CF23 5NB (GB)
(74) Representative: Fyles, Julie Marie
(86) International application number: PCT/GB2011/050298
(87) International publication number: WO 2011/107772

(56) References cited:
- KEMPIAK S J ET AL: "A neural Wiskott-Aldrich syndrome protein-mediated pathway for localized activation of actin polymerization that is regulated by cortactin", JOURNAL OF BIOLOGICAL CHEMISTRY 20050218 AMERICAN SOCIETY FOR BIOCHEMISTRY AND MOLECULAR BIOLOGY INC. US, vol. 280, no. 7, 18 February 2005 (2005-02-18), pages 5836-5842, XP002633043, DOI: DOI:10.1074/JBC.M410713200
- FERNANDO HERMAN S ET AL: "WAVE1 is associated with invasiveness and growth of prostate cancer cells.", THE JOURNAL OF UROLOGY OCT 2008 LNKD- PUBMED:18710763, vol. 180, no. 4, October 2008 (2008-10), pages 1515-1521, XP002633045, ISSN: 1527-3792
- PARK HAEIN ET AL: "Cdc42 Regulates Fc(gamma) Receptor-mediated Phagocytosis through the Activation and Phosphorylation of Wiskott-Aldrich Syndrome Protein (WASP) and Neural-WASP", MOLECULAR BIOLOGY OF THE CELL, vol. 20, no. 21, November 2009 (2009-11), pages 4500-4508, XP002633048, ISSN: 1059-1524
- INOUE T ET AL: "Effects of chemical inhibition of N-WASP, a critical regulator of actin polymerization on aqueous humor outflow through the conventional pathway", EXPERIMENTAL EYE RESEARCH, ACADEMIC PRESS LTD, LONDON, vol. 90, no. 2, 1 February 2010 (2010-02-01), pages 360-367, XP026886548, ISSN: 0014-4835, DOI: DOI:10.1016/J.EXER.2009.11.015 [retrieved on 2009-12-02]
- LORENZ M ET AL: "Imaging Sites of N-WASP Activity in Lamellipodia and Invadopodia of Carcinoma Cells", CURRENT BIOLOGY, CURRENT SCIENCE, GB, vol. 14, no. 8, 20 April 2004 (2004-04-20) , pages 697-703, XP025916309, ISSN: 0960-9822, DOI: DOI:10.1016/J.CUB.2004.04.008 [retrieved on 2004-04-20]
- SCOTT ET AL: "Ribozymes", CURRENT OPINION IN STRUCTURAL BIOLOGY, ELSEVIER LTD, GB, vol. 17, no. 3, 1 June 2007 (2007-06-01), pages 280-286, XP022144635, ISSN: 0959-440X, DOI: DOI:10.1016/J.SBI.2007.05.003
- RAMESH N ET AL: "Waltzing with WASP", TRENDS IN CELL BIOLOGY 19990101 GB LNKD- DOI:10.1016/S0962-8924(98)01411-1, vol. 9, no. 1, 1 January 1999 (1999-01-01) , pages 15-19, XP002633042, ISSN: 0962-8924
- LYUBIMOVA ANNA ET AL: "Neural Wiskott-Aldrich syndrome protein modulates Wnt signaling and is required for hair follicle cycling in mice", JOURNAL OF CLINICAL INVESTIGATION, vol. 120, no. 2, February 2010 (2010-02), pages 446-456, XP002633044, ISSN: 0021-9738
- YAMAGUCHI HIDEKI ET AL: "Molecular mechanisms of invadopodium formation: the role of the N-WASP-Arp2/3 complex pathway and cofilin", JOURNAL OF CELL BIOLOGY, vol. 168, no. 3, 31 January 2005 (2005-01-31), pages 441-452, XP002633046, ISSN: 0021-9525
- MIKI HIROAKI ET AL: "Tyrosine kinase signaling regulates Wiskott-Aldrich syndrome protein function, which is essential for megakaryocyte differentiation", CELL GROWTH AND DIFFERENTIATION, vol. 8, no. 2, 1997, pages 195-202, XP002633047, ISSN: 1044-9523
- MISRA ET AL: "N-WASP plays a critical role in fibroblast adhesion and spreading", BIOCHEMICAL AND BIOPHYSICAL RESEARCH COMMUNICATIONS, ACADEMIC PRESS INC. ORLANDO, FL, US, vol. 364, no. 4, 24 October 2007 (2007-10-24), pages 908-912, XP022344210, ISSN: 0006-291X, DOI: DOI:10.1016/J.BBRC.2007.10.086
- EMING S A ET AL: "Treatment of chronic wounds: state of the art and future concepts", CELLS TISSUES ORGANS, KARGER, BASEL, CH, vol. 172, no. 2, 1 January 2002 (2002-01-01), pages 105-117, XP008114391, ISSN: 1422-6405, DOI: DOI:10.1159/000065611
- VELNAR T ET AL: "The Wound Healing Process: an Overview of the Cellular and Molecular Mechanisms", JOURNAL OF INTERNATIONAL MEDICAL RESEARCH, vol. 37, no. 5, September 2009 (2009-09), pages 1528-1542, XP002633143, ISSN: 0300-0605
- C. J. GUERRIERO ET AL: "N-WASP inhibitor wiskostatin nonselectively perturbs membrane transport by decreasing cellular ATP levels", AJP: CELL PHYSIOLOGY, vol. 292, no. 4, 1 January 2006 (2006-01-01), pages C1562-C1566, XP55001831, ISSN: 0363-6143, DOI: 10.1152/ajpcell.00426.2006
- JIANG W G ET AL: "nWASP, the Neural Wiskott-Aldrich Syndrome Protein, Plays a Pivotal Role in the Control of Endothelial Migration and Angiogenesis", CANCER RESEARCH, vol. 69, no. 24, Suppl. 3, December 2009 (2009-12), page 643S, XP002648981, & 32ND ANNUAL SAN ANTONIO BREAST CANCER SYMPOSIUM; SAN ANTONIO, TX, USA; DECEMBER 09 -13, 2009 ISSN: 0008-5472
- J. MAGDALENA: "Involvement of the Arp2/3 Complex and Scar2 in Golgi Polarity in Scratch Wound Models", MOLECULAR BIOLOGY OF THE CELL, vol. 14, no. 2, 1 February 2003 (2003-02-01), pages 670-684, XP55001860, ISSN: 1059-1524, DOI: 10.1091/mbc.E02-06-0345
- RADHA ET AL: "C3G is required for c-Abl-induced filopodia and its overexpression promotes filopodia formation", EXPERIMENTAL CELL RESEARCH, ACADEMIC PRESS, US, vol. 313, no. 11, 15 June 2007 (2007-06-15), pages 2476-2492, XP022118263, ISSN: 0014-4827, DOI: DOI:10.1016/J.YEXCR.2007.03.019

### SEQUENCE LISTING

<110> University College Cardiff Consultants Limited
   Jiang, Wenguo
   Harding, Keith
<120> Molecular Target for Healing or Treating Wounds
<130> 0033P/EP
<150> GB1003652.3
   <151> 2010-03-05
<160> 21
<170> PatentIn version 3.5
<210> 1
   <211> 20
   <212> DNA
   <213> Homo sapiens
<400> 1
   agtccctctt cactttcctc 20
<210> 2
   <211> 20
   <212> DNA
   <213> Homo sapiens
<400> 2
   gcttttccct tcttcttttc 20
<210> 3
   <211> 20
   <212> DNA
   <213> Homo sapiens
<400> 3
   attttcatac ctttgctgga 20
<210> 4
   <211> 20
   <212> DNA
   <213> Homo sapiens
<400> 4
   taacagcttc aacacctcct 20
<210> 5
   <211> 20
   <212> DNA
   <213> Homo sapiens
<400> 5
   gagctggatg agaacaacac 20
<210> 6
   <211> 38
   <212> DNA
   <213> Homo sapiens
<400> 6
   actgaacctg accgtacaaa agaagtggca ggaagagt 38
<210> 7
   <211> 49
   <212> DNA
   <213> Homo sapiens
<400> 7
   ctgcaggagt tctttgacca catacagttc cctgatgagt ccgtgagga 49
<210> 8
   <211> 45
   <212> DNA
   <213> Homo sapiens
<400> 8
   actagttggt gcagttatat gcagcagatt tcgtcctcac ggact 45
<210> 9
   <211> 49
   <212> DNA
   <213> Homo sapiens
<400> 9
   ctgcagacaa gcaacaccac tgcacttctt tctgatgagt ccgtgagga 49
<210> 10
   <211> 52
   <212> DNA
   <213> Homo sapiens
<400> 10
   actagttata tgcagcagat cggaactgta tgtggtttcg tcctcacgga ct 52
<210> 11
   <211> 44
   <212> DNA
   <213> Homo sapiens
<400> 11
   ctgcaggtgc agctgtggga gctcttctga tgagtccgtg agga 44
<210> 12
   <211> 56
   <212> DNA
   <213> Homo sapiens
<400> 12
   actagtgcta ggggaagagg cgctcctccc ccaccacctt ttcgtcctca cggact 56
<210> 13
   <211> 505
   <212> PRT
   <213> Homo sapiens
<400> 13
   Met Ser Ser Val Gln Gln Gln Pro Pro Pro Pro Arg Arg Val Thr Asn
   1               5                   10                  15
   Val Gly Ser Leu Leu Leu Thr Pro Gln Glu Asn Glu Ser Leu Phe Thr
               20                  25                  30
   Phe Leu Gly Lys Lys Cys Val Thr Met Ser Ser Ala Val Val Gln Leu
           35                  40                  45
   Tyr Ala Ala Asp Arg Asn Cys Met Trp Ser Lys Lys Cys Ser Gly Val
       50                  55                  60
   Ala Cys Leu Val Lys Asp Asn Pro Gln Arg Ser His Phe Leu Arg Ile
   65                  70                  75                  80
   Phe Asp Ile Lys Asp Gly Lys Leu Leu Trp Glu Gln Glu Leu Tyr Asn
                   85                  90                  95
   Asn Phe Val Tyr Asn Ser Pro Arg Gly Tyr Phe His Thr Phe Ala Gly
               100                 105                 110
   Asp Thr Cys Gln Val Ala Leu Asn Phe Ala Asn Glu Glu Glu Ala Lys
           115                 120                 125
   Lys Phe Arg Lys Ala Val Thr Asp Leu Leu Gly Arg Arg Gln Arg Lys
       130                 135                 140
   Ser Glu Lys Arg Arg Asp Pro Pro Asn Gly Pro Asn Leu Pro Met Ala
   145                 150                 155                 160
   Thr Val Asp Ile Lys Asn Pro Glu Ile Thr Thr Asn Arg Phe Tyr Gly
                   165                 170                 175
   Pro Gln Val Asn Asn Ile Ser His Thr Lys Glu Lys Lys Lys Gly Lys
               180                 185                 190
   Ala Lys Lys Lys Arg Leu Thr Lys Gly Asp Ile Gly Thr Pro Ser Asn
           195                 200                 205
   Phe Gln His Ile Gly His Val Gly Trp Asp Pro Asn Thr Gly Ser Asp
       210                 215                 220
   Leu Asn Asn Leu Asp Pro Glu Leu Lys Asn Leu Phe Asp Met Cys Gly
   225                 230                 235                 240
   Ile Leu Glu Ala Gln Leu Lys Glu Arg Glu Thr Leu Lys Val Ile Tyr
                   245                 250                 255
   Asp Phe Ile Glu Lys Thr Gly Gly Val Glu Ala Val Lys Asn Glu Leu
   260 265 270
   Arg Arg Gln Ala Pro Pro Pro Pro Pro Pro Ser Arg Gly Gly Pro Pro
   275 280 285
   Pro Pro Pro Pro Pro Pro His Ser Ser Gly Pro Pro Pro Pro Pro Ala
   290 295 300
   Arg Gly Arg Gly Ala Pro Pro Pro Pro Pro Ser Arg Ala Pro Thr Ala
   305 310 315 320
   Ala Pro Pro Pro Pro Pro Pro Ser Arg Pro Ser Val Glu Val Pro Pro
   325 330 335
   Pro Pro Pro Asn Arg Met Tyr Pro Pro Pro Pro Pro Ala Leu Pro Ser
   340 345 350
   Ser Ala Pro Ser Gly Pro Pro Pro Pro Pro Pro Ser Val Leu Gly Val
   355 360 365
   Gly Pro Val Ala Pro Pro Pro Pro Pro Pro Pro Pro Pro Pro Pro Gly
   370 375 380
   Pro Pro Pro Pro Pro Gly Leu Pro Ser Asp Gly Asp His Gln Val Pro
   385 390 395 400
   Thr Thr Ala Gly Asn Lys Ala Ala Leu Leu Asp Gln Ile Arg Glu Gly
   405 410 415
   Ala Gln Leu Lys Lys Val Glu Gln Asn Ser Arg Pro Val Ser Cys Ser
   420 425 430
   Gly Arg Asp Ala Leu Leu Asp Gln Ile Arg Gln Gly Ile Gln Leu Lys
   435 440 445
   Ser Val Ala Asp Gly Gln Glu Ser Thr Pro Pro Thr Pro Ala Pro Thr
   450 455 460
   Ser Gly Ile Val Gly Ala Leu Met Glu Val Met Gln Lys Arg Ser Lys
   465 470 475 480
   Ala Ile His Ser Ser Asp Glu Asp Glu Asp Glu Asp Asp Glu Glu Asp
   485 490 495
   Phe Glu Asp Asp Asp Glu Trp Glu Asp
   500 505
<210> 14
   <211> 1792
   <212> DNA
   <213> Homo sapiens
<400> 14
   ggcagaggga caacgaccat ccggccctag cctggccggg cgggtgccgg gagcttccct 60
   ttctcagcgc ggcggaaggt ggctcgccgt cagcgcctgc ttccctcgac ctcgtcctcc 120
   tccccgctcc ggacgagccg agatgtggcg cctctgactc cacttctccc cgcccctgtc 180
   accgagaggg ggaacgagct ctcgcccact cgccggagag acggccctgg actcccaacc 240
   ccgccggcga aaccatgagc tccgtccagc agcagccgcc gccgccgcgg agggtcacca 300
   acgtggggtc cctgttgctc accccgcagg agaacgagtc cctcttcact ttcctcggca 360
   agaaatgtgt gactatgtct tcagcagtgg tgcagttata tgcagcagat cggaactgta 420
   tgtggtcaaa gaagtgcagt ggtgttgctt gtcttgttaa ggacaatcca cagagatctc 480
   attttttaag aatatttgac attaaggatg ggaaactatt gtgggaacaa gagctataca 540
   ataactttgt atataatagt cctagaggat attttcatac ctttgctgga gatacttgtc 600
   aagttgctct taattttgcc aatgaagaag aagcaaaaaa atttcgaaaa gcagttacag 660
   accttttggg ccgtcgacaa aggaaatctg agaaaagacg agatccccca aatggtccta 720
   atctacccat ggctacagtt gatataaaaa atccagaaat cacaacaaat agattttatg 780
   gtccacaagt caacaacatc tcccatacca aagaaaagaa gaagggaaaa gctaaaaaga 840
   agagattaac caagggagat ataggaacac caagcaattt ccagcacatt ggacatgttg 900
   gttgggatcc aaatacaggc tctgatctga ataatttgga tccagaattg aagaatcttt 960
   ttgatatgtg tggaatctta gaggcacaac ttaaagaaag agaaacatta aaagttatat 1020
   atgactttat tgaaaaaaca ggaggtgttg aagctgttaa aaatgaactg cggaggcaag 1080
   caccaccacc tccaccacca tcaaggggag ggccacctcc tcctcctccc cctccacata 1140
   gctcgggtcc tcctcctcct cctgctaggg gaagaggcgc tcctccccca ccaccttcaa 1200
   gagctcccac agctgcacct ccaccaccgc ctccttccag gccaagtgta gaagtccctc 1260
   caccaccgcc aaataggatg taccctcctc cacctccagc ccttccctcc tcagcacctt 1320
   cagggcctcc accaccacct ccatctgtgt tgggggtagg gccagtggca ccacccccac 1380
   cgcctccacc tccacctcct cctgggccac cgcccccgcc tggcctgcct tctgatgggg 1440
   accatcaggt tccaactact gcaggaaaca aagcagctct tttagatcaa attagagagg 1500
   gtgctcagct aaaaaaagtg gagcagaaca gtcggccagt gtcctgctct ggacgagatg 1560
   cactgttaga ccagatacga cagggtatcc aactaaaatc tgtggctgat ggccaagagt 1620
   ctacaccacc aacacctgca cccacttcag gaattgtggg tgcattaatg gaagtgatgc 1680
   agaaaaggag caaagccatt cattcttcag atgaagatga agatgaagat gatgaagaag 1740
   attttgagga tgatgatgag tgggaagact gatctatata ttatatatat ac 1792
<210> 15
   <211> 502
   <212> PRT
   <213> Homo sapiens
<400> 15
<210> 16
   <211> 1796
   <212> DNA
   <213> Homo sapiens
<400> 16
   gagaagacaa gggcagaaag caccatgagt gggggcccaa tgggaggaag gcccgggggc 60
   cgaggagcac cagcggttca gcagaacata ccctccaccc tcctccagga ccacgagaac 120
   cagcgactct ttgagatgct tggacgaaaa tgcttgacgc tggccactgc agttgttcag 180
   ctgtacctgg cgctgccccc tggagctgag cactggacca aggagcattg tggggctgtg 240
   tgcttcgtga aggataaccc ccagaagtcc tacttcatcc gcctttacgg ccttcaggct 300
   ggtcggctgc tctgggaaca ggagctgtac tcacagcttg tctactccac ccccaccccc 360
   ttcttccaca ccttcgctgg agatgactgc caagcggggc tgaactttgc agacgaggac 420
   gaggcccagg ccttccgggc cctcgtgcag gagaagatac aaaaaaggaa tcagaggcaa 480
   agtggagaca gacgccagct acccccacca ccaacaccag ccaatgaaga gagaagagga 540
   gggctcccac ccctgcccct gcatccaggt ggagaccaag gaggccctcc agtgggtccg 600
   ctctccctgg ggctggcgac agtggacatc cagaaccctg acatcacgag ttcacgatac 660
   cgtgggctcc cagcacctgg acctagccca gctgataaga aacgctcagg gaagaagaag 720
   atcagcaaag ctgatattgg tgcacccagt ggattcaagc atgtcagcca cgtggggtgg 780
   gacccccaga atggatttga cgtgaacaac ctcgacccag atctgcggag tctgttctcc 840
   agggcaggaa tcagcgaggc ccagctcacc gacgccgaga cctctaaact tatctacgac 900
   ttcattgagg accagggtgg gctggaggct gtgcggcagg agatgaggcg ccaggagcca 960
   cttccgccgc ccccaccgcc atctcgagga gggaaccagc tcccccggcc ccctattgtg 1020
   gggggtaaca agggtcgttc tggtccactg ccccctgtac ctttggggat tgccccaccc 1080
   ccaccaacac cccggggacc cccaccccca ggccgagggg gccctccacc accaccccct 1140
   ccagctactg gacgttctgg accactgccc cctccacccc ctggagctgg tgggccaccc 1200
   atgccaccac caccgccacc accgccaccg ccgcccagct ccgggaatgg accagcccct 1260
   cccccactcc ctcctgctct ggtgcctgcc gggggcctgg cccctggtgg gggtcgggga 1320
   gcgcttttgg atcaaatccg gcagggaatt cagctgaaca agacccctgg ggccccagag 1380
   agctcagcgc tgcagccacc acctcagagc tcagagggac tggtgggggc cctgatgcac 1440
   gtgatgcaga agagaagcag agccatccac tcctccgacg aaggggagga ccaggctggc 1500
   gatgaagatg aagatgatga atgggatgac tgagtggctg agttacttgc tgccctgtgc 1560
   tcctccccgc aggacatggc tccccctcca cctgctctgt gcccaccctc cactctcctc 1620
   ttccaggccc ccaacccccc atttcttccc caccaacccc tccaatgctg ttatccctgc 1680
   ctggtcctca cactcaccca acaatcccaa ggcccttttt atacaaaaat tctcagttct 1740
   cttcactcaa ggatttttaa agaaaaataa aagaattgtc tttctgtctc tctata 1796
<210> 17
   <211> 83
   <212> DNA
   <213> Homo sapiens
<400> 17
   ctgcaggagt tctttgacca catacagttc cctgatgagt ccgtgaggac gaaatctgct 60
   gcatataact gcaccacact agt 83
<210> 18
   <211> 89
   <212> DNA
   <213> Homo sapiens
<400> 18
   ctgcagacaa gcaacaccac tgcacttctt tctgatgagt ccgtgaggac gaaaccacat 60
   acagttccga tctgctgcat ataactagt 89
<210> 19
   <211> 87
   <212> DNA
   <213> Homo sapiens
<400> 19
   ctgcaggtgc agctgtggga gctcttctga tgagtccgtg aggacgaaaa ggtggtgggg 60
   gaggagcgcc tcttccccta gcctagt 87
<210> 20
   <211> 18
   <212> DNA
   <213> Homo sapiens
<400> 20
   atgagctccg tccagcag 18
<210> 21
   <211> 21
   <212> DNA
   <213> Homo sapiens
<400> 21
   tcagtcttcc cactcatcat c 21

## Description

### Field of the Invention

The present invention relates to at least one molecular target for healing or treating wounds and, in particular, human wounds. More particularly still, the molecular target has application in the treatment of chronic wounds. Further, the invention concerns a novel therapeutic for treating said wounds and a novel gene therapy approach, involving said molecular target, for treating said wounds.

### Background of the Invention

In one form or another, chronic and poorly healing wounds constitute a major burden on the UK health system. Moreover, in certain member countries of the EU health expenses relating to wound healing are already approaching the third most expensive drain on health care funding.

Chronic foot ulcers are a major complication of diabetes, accounting for up to 25% of all hospital admissions involving diabetes, and at a cost to the UK National Health Service of £250M annually. Chronic foot ulcers cause substantial morbidity, impair the quality of life, and are the major cause of lower limb amputation. Despite careful attention to foot care, as many as 25% of diabetics develop foot ulcers in their lifetimes. The causes of lower limb ulceration are the same in diabetics as in non-diabetics, namely neuropathy, ischaemia and trauma. However, this "pathogenic triad" predisposes wounds to infection, which can also contribute to the non-healing nature of the wounds.

Current treatment involves removing pressure from the area, debridement, wound dressing and management of infection: surgical resection and vascular reconstruction may be required in more advanced disease, which ultimately may necessitate amputation.

In addition to lower limb ulcers in diabetics, another major resource health cost is created by pressure wounds or ulcers that result, for example, from failure to provide routine nursing or medical care. In the UK 412,000 people are affected annually by this sort of wound at a cost of £1.4-2.1 billion.

The healing of a wound is controlled by complex biological processes that involve a diverse number of cell types; complex interactions between cells and tissues; the activation of the immune system and the activation of the angiogenic process. Moreover, all of these processes involve a large number of molecules.

A typical healing process can be divided into 5 distinct, but closely related, stages: clotting stage, acute inflammation stage, matrix deposition stage, capillary formation stage and re-epithelialisation stage. A diverse number of factors are involved in and control each of these stages. Deficiencies in any aspect of the process may result in defective wound healing. Thus, a 'normal' healing process may be defective as a result of either intrinsic or external factors, which manifest as 'abnormal non-healing' or 'chronic' wounds. It is these chronic or 'non-healing' wounds that present the greatest challenge to the quality of a patient's life and mounting expenses to the healthcare system.

Although some common clinical/pathological factors may assist in pre-judging if a wound may be 'healing' or 'non-healing', or if an acute wound may become chronic, there is no specific laboratory test(s) to distinguish wound type. Additionally, there is no clear way to define how to predict the healing process and a patient's likely response to treatment in chronic wound care.

### WASP/n-WASP

Wiskott-Aldrich syndrome (WAS), also described as Werlhof's disease (Van den Bosch and Drukker 1964), was originally described in American kindred where it was manifested as eczema, thrombocytopenia, proneness to infection, and bloody diarrhoea (Alfrich et al. 1954). Death usually occurs before the age of 10 years. The causes of death are mainly infections or bleeding, but also development of malignancies: lymphoreticular tumors and leukemia reticuloendothelial system malignancies (Perry et al. 19980, Ten Bense et al 1966, Sullivan et al. 1994).

It has been recognised that the Wiskott-Aldrich protein provides a link between Cell Division Cycle 42 (Cdc42) and the actin cytoskeleton (Symons et al. 1996). T lymphocytes of affected males with WAS exhibit a severe disturbance of the actin cytoskeleton, suggesting that the WAS protein may regulate its organization. The WAS protein interacts with Cdc42, a member of the RHO family of GTPases (thus GTP-dependent) it was detected in cell lysates, in transient transfections, and with purified recombinant proteins (Kolluri et al. 1996) suggesting that the WAS protein functions as a signal transduction adaptor downstream of Cdc42, and that cytoskeletal abnormalities may result from a defect in Cdc42 signalling. It has since been demonstrated that WAS is a rare X-linked disorder with variable clinical phenotypes that correlate with the type of mutations in the WAS protein (WASP) gene (Ochs and Thrasher 2006).

It has also been shown that WASP is a key regulator of actin polymerization in hematopoietic cells with 5 domains involved in signalling, cell motility/migration, in immune synapse formation and in facilitating the nuclear translocation of nuclear factor kappaB (Ochs and Thrasher 2006). Mutations of WASP are located throughout the gene and either inhibit or dysregulate normal WASP function: classic WAS occurs when WASP is absent; X-linked thrombocytopenia when mutated WASP is expressed; and X-linked neutropenia when missense mutations occur in the Cdc42-binding site (Ochs and Thrasher 2006).

Miki et al (1996) first described a 65 kDa protein from brain that bound to the SH3 domains of Ash/Grb2. The amino acid sequence was approximately 50% homologous to Wiskott-Aldrich syndrome protein (WASP) and was termed N-WASP (neural-WASP). N-WASP has several functional motifs (such as a pleckstrin homology (PH) domain and cofilin-homologous region) through which N-WASP depolymerizes actin filaments. N-WASP-stimulated actin assembly is responsible for membrane ruffling (Zalevsky et al., 2001), a process that actively involves the cytoskeletal associated protein family, ERM (ezrin-moesin-radixin)(Brescher et al 1989, Hiscox and Jiang 1999). N-WASP activity is regulated by an intramolecular interaction that is alleviated following concomitant binding of Cdc42-GTP to the Cdc42/Rac interactive binding (CRIB) domain and Ptdlns(4,5)*P*₂ to the polybasic region (Kovacs et al. 2006). We have recently reported that two major complexes that linked to the WASP family, namely the ERM family and Rho GTPases were aberrantly expressed in human breast cancer (Harrison et al 2003, Jiang et al 2003).

Thus the two homologous proteins share a common functionality in stimulating actin assembly or organisation of the cytoskeleton.

In our investigations we have surprisingly discovered what may be termed a loss in the control of nWASP activities in chronic, or abnormal, wounds where nWASP is over-expressed compared with acute wounds which have low levels of nWASP expression. This observation indicated to us the potential role of nWASP or nWASP-like proteins in the healing process. We therefore undertook work to block the aberrantly expressed nWASP in chronic wounds and found it helped in the healing of keratinocytes. Further work showed that manipulating nWASP is an effective and safe way to promote the healing of difficult wounds where nWASP is over-expressed and this over-expression results in a hindered healing process.

In summary, we have identified at least one molecular target for treating wounds and in particular human wounds. More particularly, but not exclusively, said molecular target has application in the treatment of chronic wounds. The molecular target is nWASP and therefore the invention relates to a novel therapeutic comprising an inhibitor of either, or both, nWASP expression or nWASP activity. In the former instance, the invention involves a novel gene therapy approach and in the latter instance a novel protein therapy approach. Further, given the sequence homology and structure and function homology between n-WASP and WASP the teaching of the invention further extends to WASP. Accordingly, the teaching also relates to a novel therapeutic comprising an inhibitor of either, or both, WASP expression or WASP activity. In the former instance, the invention involves a novel gene therapy approach and in the latter instance a novel protein therapy approach.

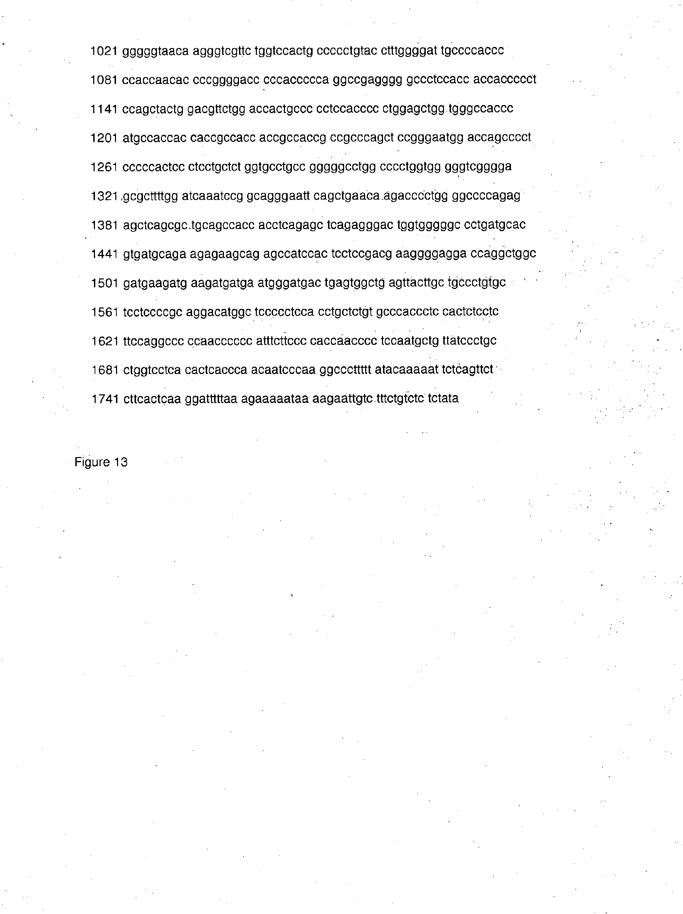

Reference herein to nWASP, or WASP, is reference to a gene or protein whose identity is shown in Figure 13.

Our invention can improve the quality of a patient's life by ensuring that new wounds do not deteriorate into a chronic state and existing chronic wounds can be treated in a way that actively promotes healing.

### Detailed description

Accordingly, in one aspect of the invention there is provided a novel therapeutic comprising an inhibitor of either, or both, nWASP gene expression or nWASP protein activity for use in the treatment of mammalian chronic wounds.

In the former instance, the invention involves a novel gene therapy approach and in the latter instance a novel protein therapy approach. Thus, in one embodiment the novel therapeutic comprises an inhibitor of nWASP gene expression, this inhibitor can be either anti-sense DNA or RNA, siRNA, or ribozymes, either naked or in the form of plasmid and viral vectors. Those skilled in the art are aware of the aforementioned inhibitory molecules and so would be able to work the invention once they knew that over-expression of nWASP contributed to the chronic wound phenotype. However, in another embodiment the novel therapeutic comprises an inhibitor of nWASP protein function, this inhibitor can be either a nWASP binding agent that binds, either reversibly or irreversibly, to inhibit protein function such as an antibody or a known, or synthesized, nWASP antagonist; or an agent that works upstream or downstream of the nWASP signalling mechanism to inhibit nWASP signalling and so negate the effects of over-expression of nWASP protein in chronic wound tissue. Those skilled in the art are aware of the aforementioned inhibitory molecules and so would be able to work the invention once they knew that over-expression of nWASP contributed to the chronic wound phenotype.

Additionally, or alternatively, the teaching herein also comprises a novel therapeutic comprising an inhibitor of either, or both, WASP gene expression or WASP protein activity for use in the treatment of mammalian chronic wounds.

As above, in the former instance, the teaching involves a novel gene therapy approach and in the latter instance a novel protein therapy approach. Thus, in one teaching the novel therapeutic comprises an inhibitor of WASP gene expression, this inhibitor can be either anti-sense DNA or RNA, siRNA, or ribozymes, either naked or in the form of plasmid and viral vectors. Those skilled in the art are aware of the afore inhibitory molecules and so would be able to use the teaching once they knew that over-expression of WASP contributed to the chronic wound phenotype. However, in another teaching the novel therapeutic comprises an inhibitor of WASP protein function, this inhibitor can be either a WASP binding agent that binds, either reversibly or irreversibly, to inhibit protein function such as an antibody or a known, or synthesized, WASP antagonist; or an agent that works upstream or downstream of the WASP signalling mechanism to inhibit WASP signalling and so negate the effects of over-expression of WASP protein in chronic wound tissue. Those skilled in the art are aware of the aforementioned inhibitory molecules and so would be able to use the teaching once they knew that over-expression of WASP contributed to the chronic wound phenotype.

In a preferred embodiment of the invention the therapeutic comprises an nWASP gene inhibitor such as transgene 1 or transgene 2 or transgene 3 described herein. These molecules are termed anti-nWASP ribozyme/RNA transgenes. Transgene 1 is produced by transcription of the nWASP gene using the following short oligos:
nWASPRib1F (5'Ctgcaggagttctttgaccacatacagttccctgatgagtccgtgagga'3) and
nWASPRib1R (5'Actagttggtgcagttatatgcagcag**A**tttcgtcctcacggact'3).

Transgene 2 is produced by transcription of the nWASP gene using the following short oligos:
nWASPrib2F (5'Ctgcagacaagcaacaccactgcacttctttctgatgagtccgtgagga'3) and
nWASPRib2R (5'Actagttatatgcagcag**ATC**ggaactgtatgtg**G**tttcgtcctcacggact'3).

Transgene 3 is produced by transcription of the nWASP gene using the following short oligos:
nWASPRib3F (5'Ctgcaggtgcagctgtgggagctcttctgatgagtccgtgagga'3) and
nWASPRib3R (5'Actagtgctaggggaagaggcgctcctcccccaccacc**T**tttcgtcctcacggact'3).

These products are antisense-hammerhead ribozyme also known as antisense-hammerhead RNA, ideally they are flanked by selected restriction sites such as pstI and SpeI and more ideally still they are cloned into a cloning vector such as pEF6/V5/His TOPO.

The sequence structure of transgene 1 is:
5'Ctgcaggagttctttgaccacatacagttccctgatgagtccgtgaggacgaaatctgctgcatataactgca ccacactagt'3

The sequence structure of transgene 2 is:
5'Ctgcagacaagcaacaccactgcacttctttctgatgagtccgtgaggacgaaaccacatacagttccgatc tgctg catataactagt'3

The sequence structure of transgene 3 is:
5'Ctgcaggtgcagctgtgggagctcttctgatgagtccgtgaggacgaaaaggtggtgggggaggagcgcc tcttcccctagcctagt'3

In a preferred embodiment of the invention the therapeutic comprises a commercially available nWASP protein inhibitor such as, without limitation, Wiskostatin (Merck Pharmaceuticals) or 187-1 (TOCRIS).

In a further teaching the therapeutic comprises a commercially available WASP protein inhibitor.

The therapeutic of the invention is for use in treating mammalian chronic wounds, ideally human.

An antibody for use in the invention is most ideally a monoclonal antibody or a humanised antibody.

In the above aspects and embodiments of the invention the therapeutic is formulated for topical application.

Alternatively, in the above aspects and embodiments of the invention the therapeutic is formulated for oral application.

Alternatively again, in the above aspects and embodiments of the invention the therapeutic is formulated for application to a dressing or impregnation in a dressing.

The therapeutic of the invention may be administered in combination with an antibiotic or antibacterial agent. Numerous such agents are known and suitable choices will be familiar to skilled practitioners.

In yet another aspect of the invention, there is provided a pharmaceutical composition for use in the treatment of mammalian chronic wounds comprising a therapeutic of the invention together with a pharmaceutically acceptable carrier.

Other active materials may also be present in the paharmaceutical composition, as may be considered appropriate or advisable for the wound being treated. For example, the composition may also contain an emollient, or the like.

The carrier, or, if more than one be present, each of the carriers, must be acceptable in the sense of being compatible with the other ingredients of the formulation and not deleterious to the recipient.

The formulations include those suitable for topical (including eye drops), oral (including buccal and sublingual), rectal, nasal or vaginal administration and may be prepared by any methods well known in the art of pharmacy.

The composition may be prepared by bringing into association the therapeutic of the invention and the carrier. In general, the formulations are prepared by uniformly and intimately bringing into association the active agent with liquid carriers or finely divided solid carriers or both, and then if necessary shaping the product. The disclosure extends to methods for preparing a pharmaceutical composition comprising bringing a therapeutic of the invention in conjunction or association with a pharmaceutically or veterinarily acceptable carrier or vehicle.

For topical application to the skin, compounds of conventional use may be made up into a cream, ointment, jelly, solution or suspension etc. Cream or ointment formulations that may be used for the composition are conventional formulations well known in the art, for example, as described in standard text books of pharmaceutics such as the British Pharmacopoeia.

Formulations for oral administration in the present invention may be presented as: discrete units such as capsules, sachets or tablets each containing a predetermined amount of the active agent; as a powder or granules; as a solution or a suspension of the active agent in an aqueous liquid or a nonaqueous liquid; or as an oil-in-water liquid emulsion or a water in oil liquid emulsion; or as a bolus etc.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

Other formulations suitable for oral administration include lozenges comprising the active agent in a flavoured or inert base base and mouthwashes comprising the active agent in a suitable liquid carrier.

There is taught herein a method for treating a mammalian wound, typically a chronic wound, which method comprises:
administering to said wound a therapeutic that inhibits either, or both of, nWASP gene expression or nWASP protein activity.

There is also taught herein another novel method for treating a mammalian wound, typically a chronic wound, which method comprises:
administering to said wound a therapeutic that inhibits either, or both of, WASP gene expression or WASP protein activity.

According to yet a further aspect of the invention there is provided a kit for use in the treatment of mammalian chronic wounds, preferably a chronic wound, wherein said kit comprises:
(a) at least one therapeutic as above described; and
(b) at least one dressing for applying to said wound.

The teaching herein also extends to a combination therapeutic comprising an inhibitor of n-WASP gene expression and an inhibitor of WASP gene expression.

Further the teaching herein extends to a combination therapeutic comprising an inhibitor of n-WASP protein activity and an inhibitor of WASP protein activity.

According to a yet further aspect of the invention there is provided a combination therapeutic for use in the treatment of mammalian chronic wounds comprising an inhibitor of: a) n-WASP gene expression; and an inhibitor of b) n-WASP protein activity.

According to a further aspect of the invention there is provided a therapeutic comprising an inhibitor of nWASP, or a homologoue thereof.

According to a further aspect of the invention there is provided use of an inhibitor of nWASP, or a homologoue thereof, in the manufacture of a medicament for treating a mammalian chronic wound.

According to a further aspect of the invention there is provided use of an inhibitor of nWASP, or a homologue thereof, for treating a mammalian chronic wound.

The term "homologue" as used herein refers to amino acid sequences which have a sequence at least 90% homologous to the amino acid sequence of nWASP and which retain the biological activity of the nWASP sequence. It is preferred that homologues are at least 90% homologous to the nWASP peptide sequence and, in increasing order of preference, at least 90%, 95% or 99% homologous to the nWASP peptide sequence.

Treatment of a wound described herein includes reference to human or veterinary use.

In the claims which follow and in the preceding description of the invention, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

Preferred features of each aspect of the invention may be as described in connection with any of the other aspects.

Other features of the present invention will become apparent from the following examples.

Moreover, unless stated otherwise, any feature disclosed herein may be replaced by an alternative feature serving the same or a similar purpose.

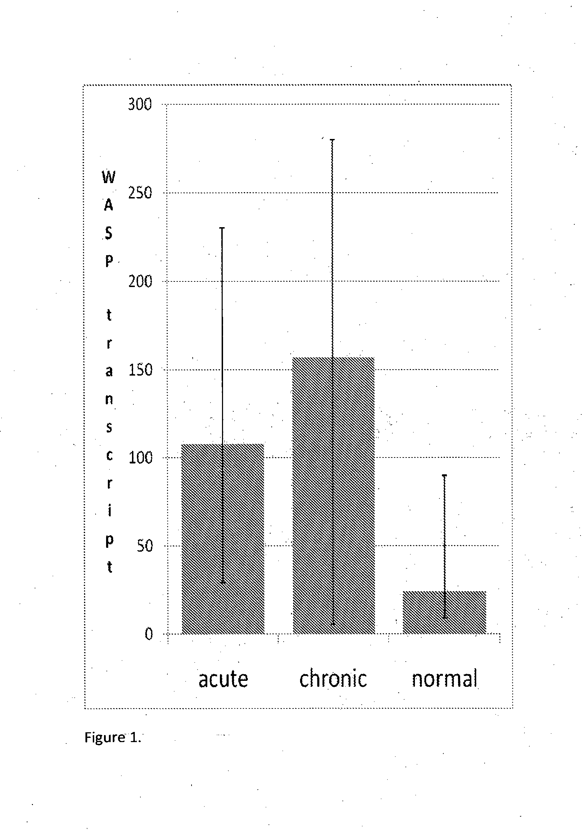

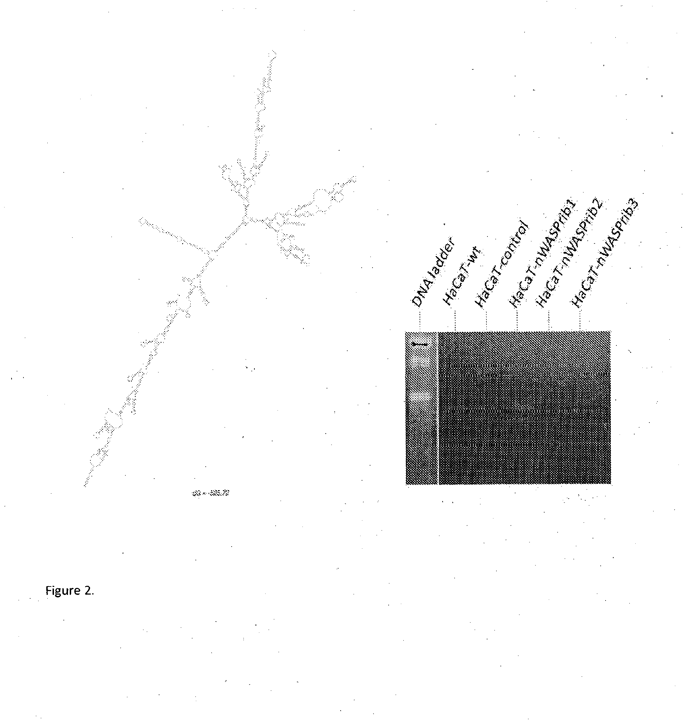

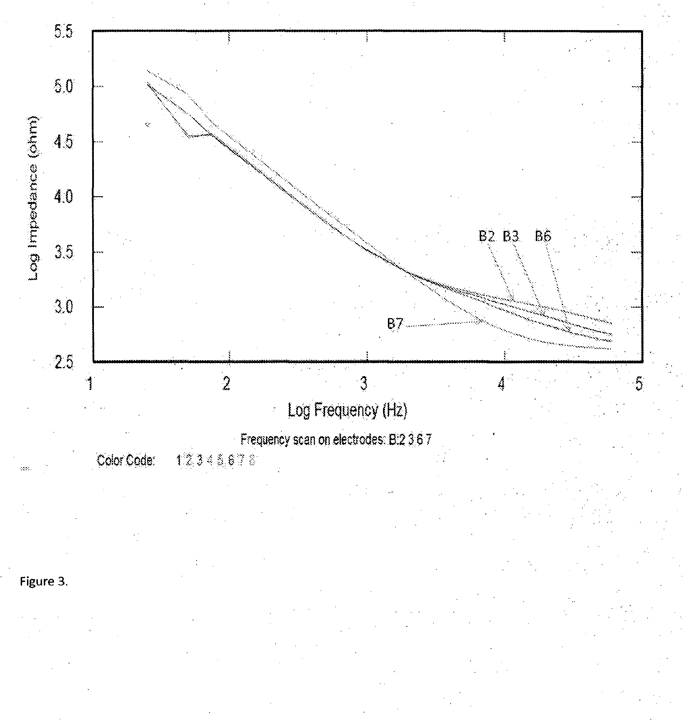

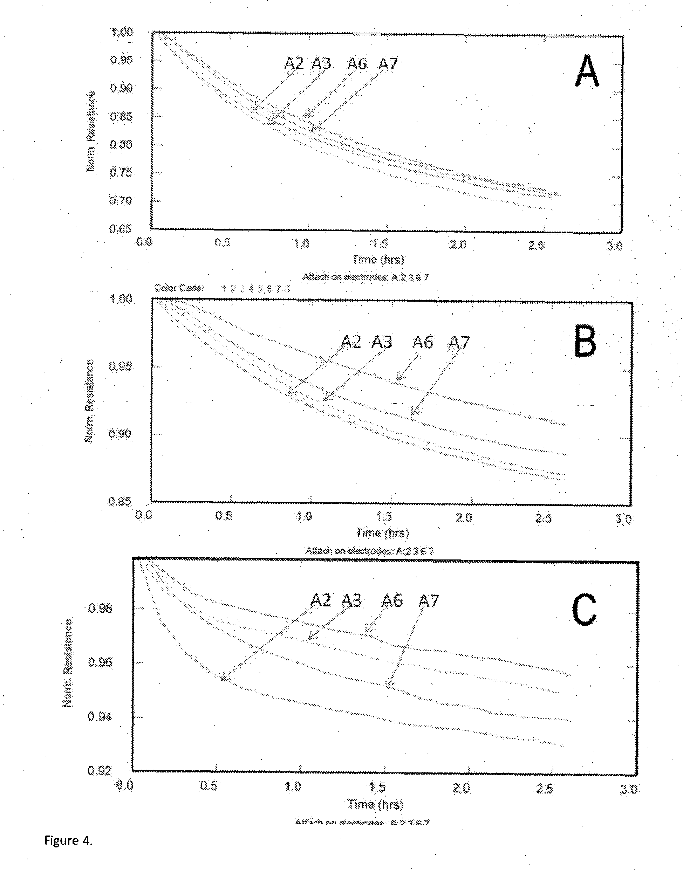

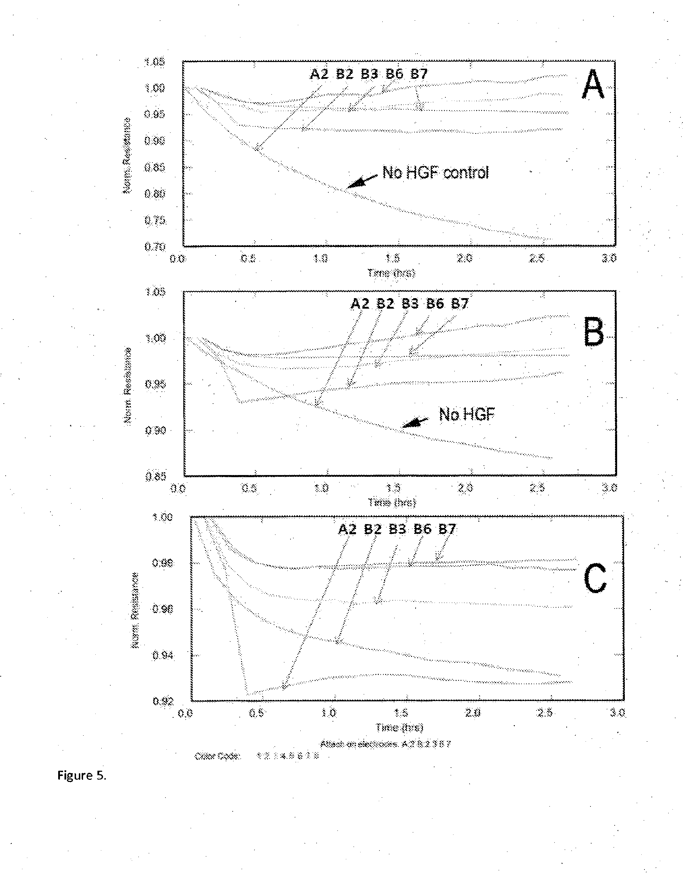

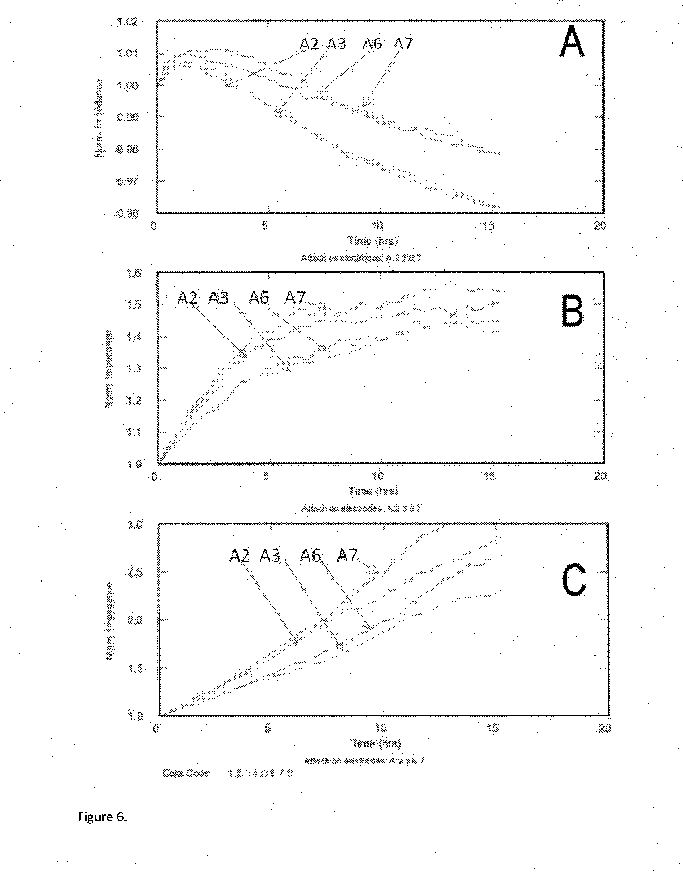

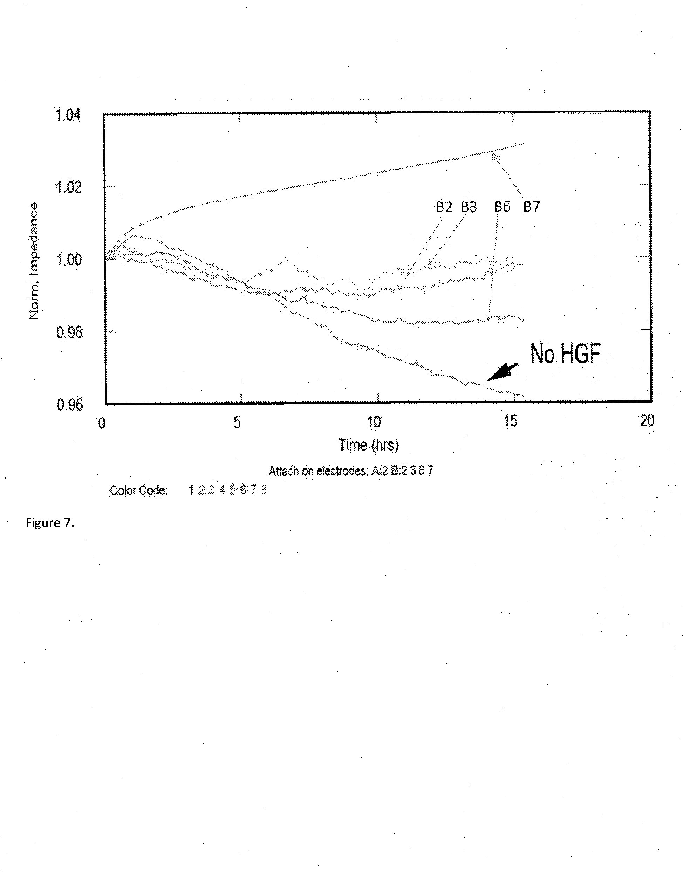

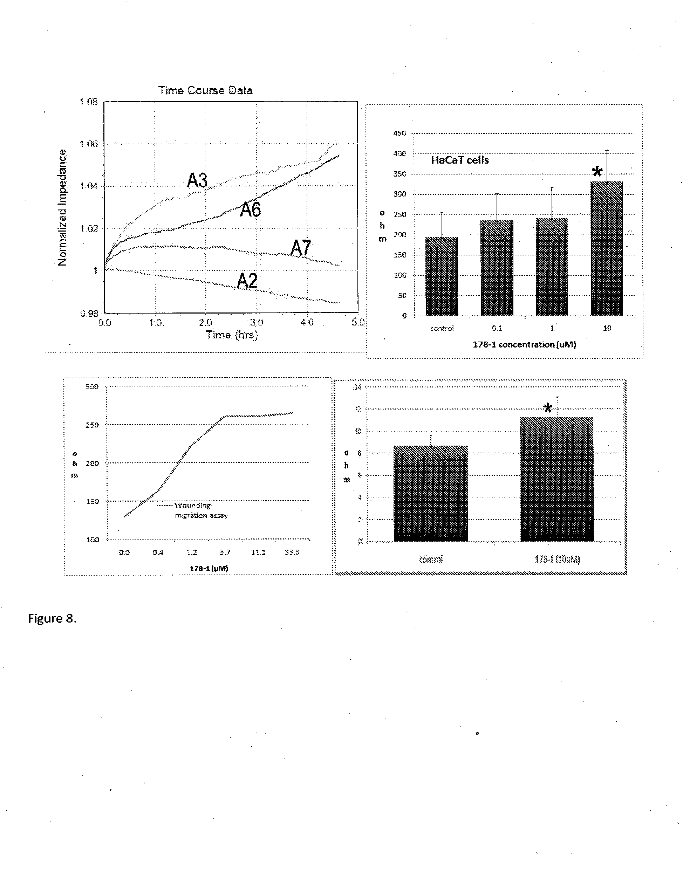

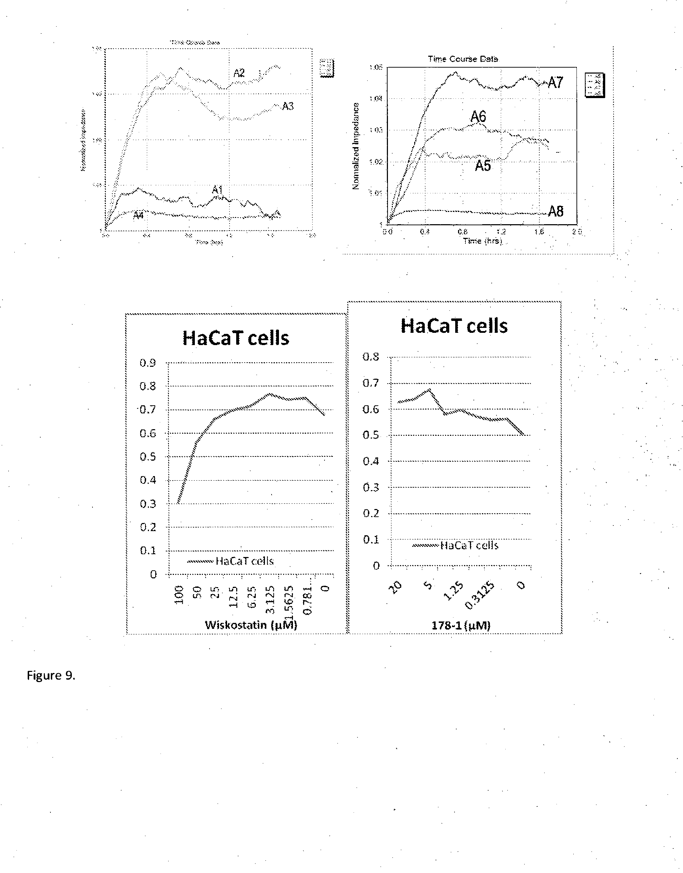

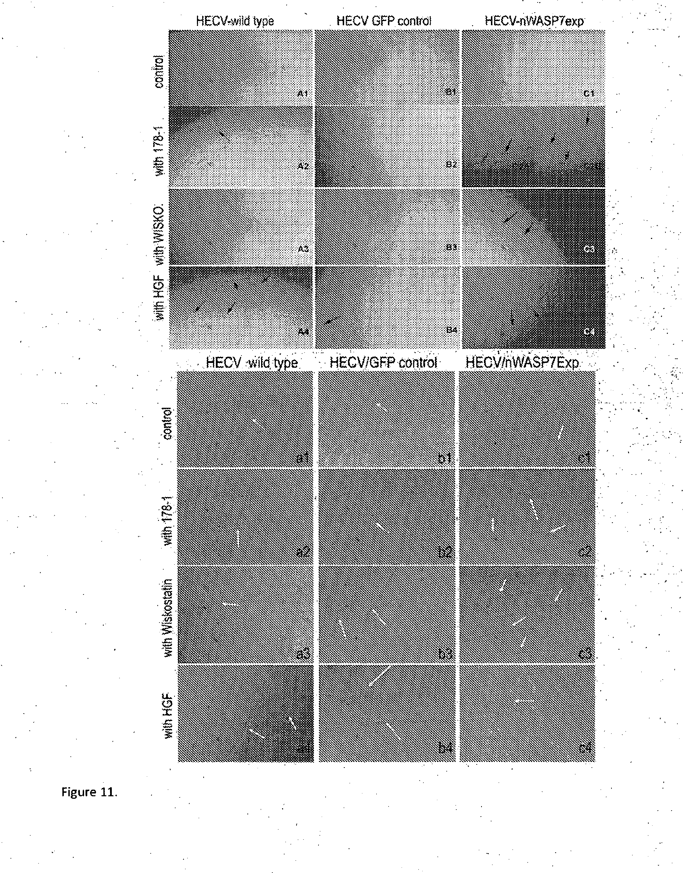

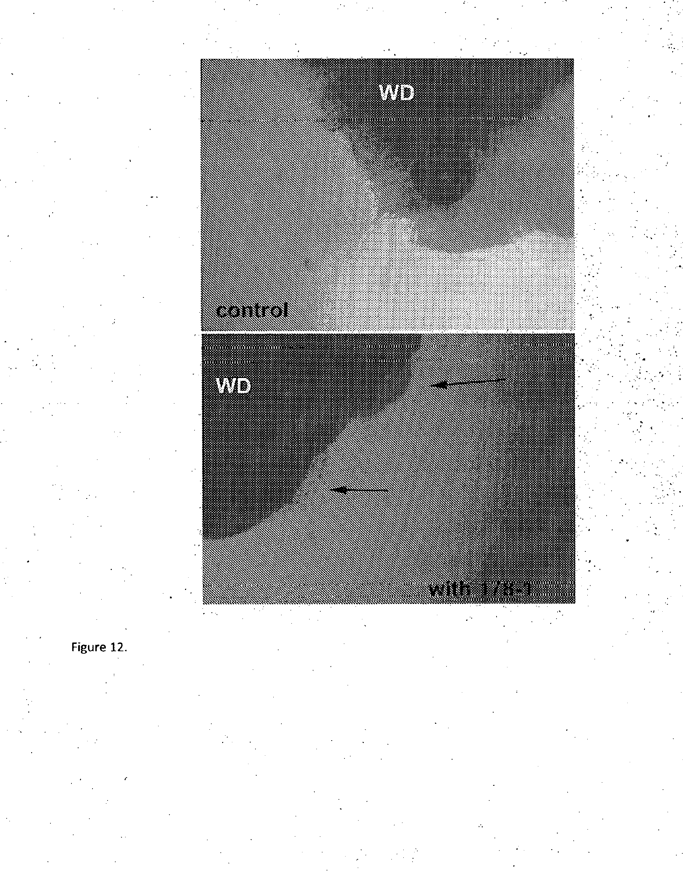

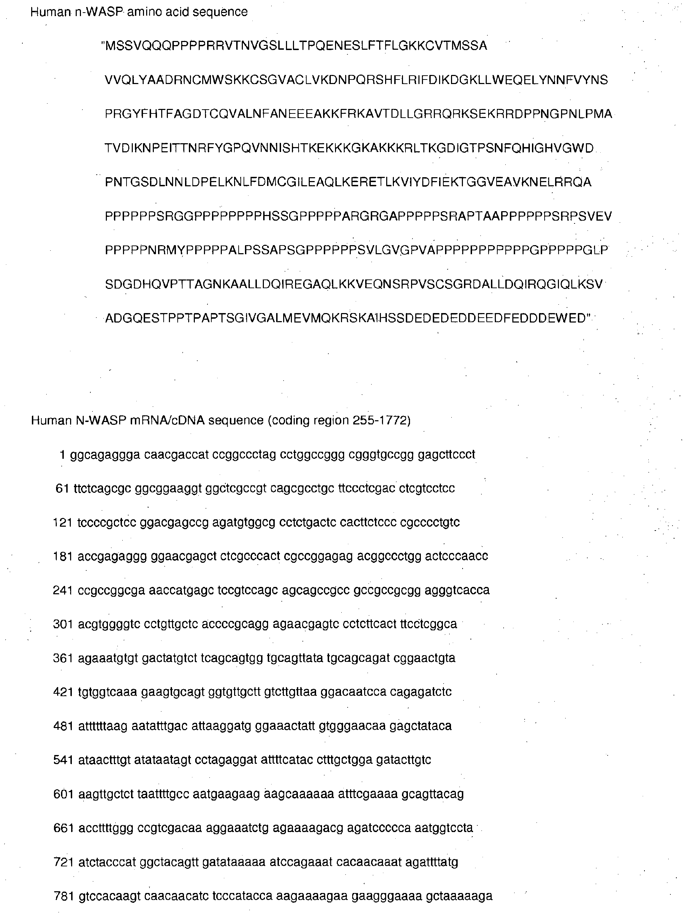

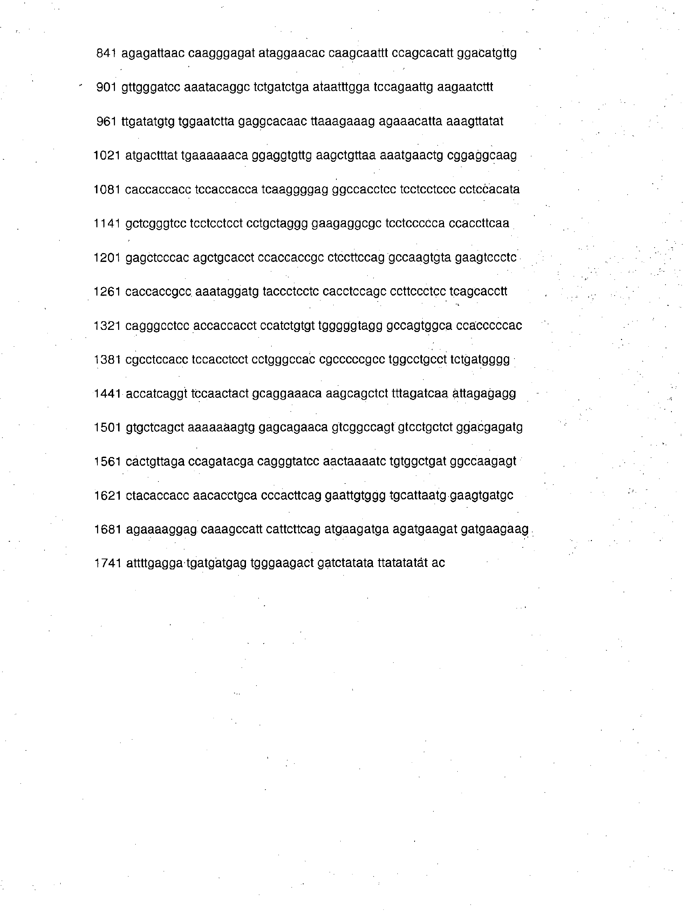

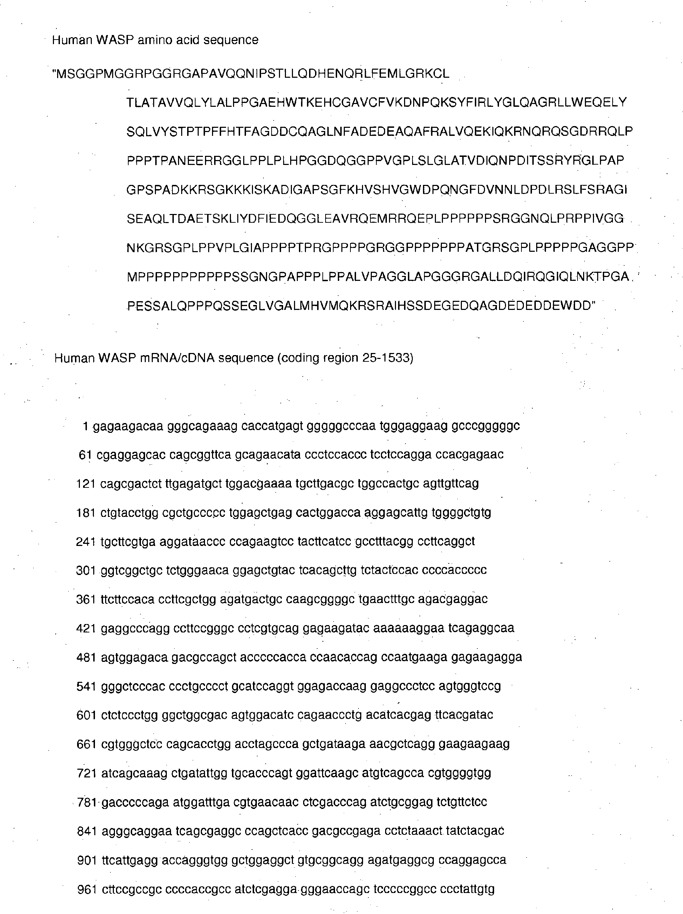

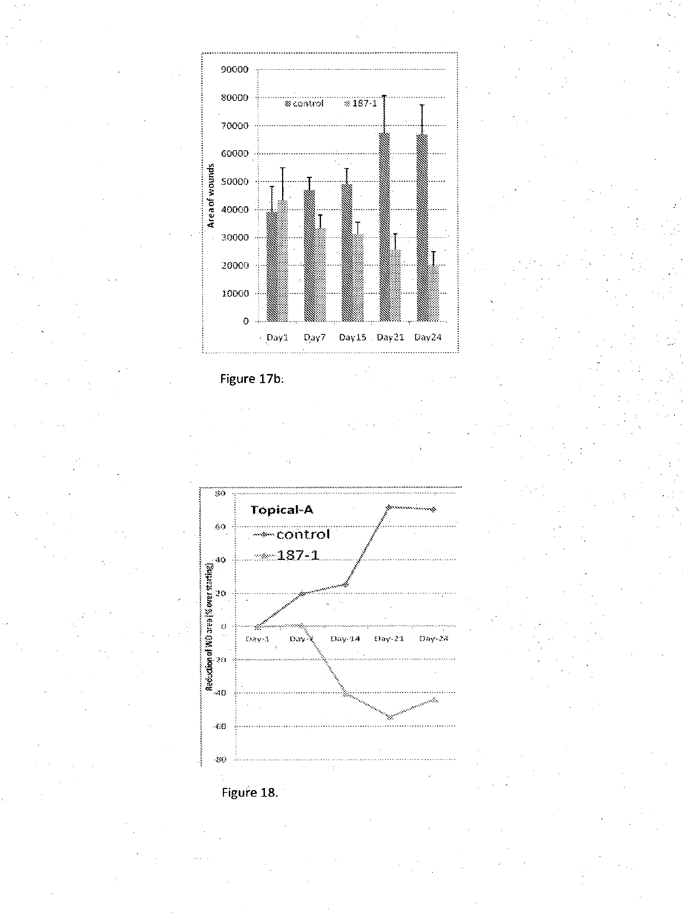

The present invention will now be described by way of the following examples with particular reference to Figures 1-21 wherein:
Figure-1. Shows expression analysis of nWASP transcript in normal skin, acute wound and abnormal/chronic wound tissues. Shown are median and the inter-quartile range of the transcript level;
Figure-2. Shows Left: The predicted secondary structure of human nWASP which was the basis for designing the anti-nWASP rizozyme transgenes. Right: The effect of anti-nWASP transgenes on levels of nWASP mRNA after transfection. Transgenes-1 2 and 3 were active in knocking down the nWASP transcript from HaCaT cells;
Figure-3. Shows frequency scanning of HaCat cells. Probe-A2: medium control; probe-B3: Wiskostatin 100nM; probe-B6: Wiskostatin 10nM; probe-B7: Wiskostatin 1 nM;
Figure-4. Shows effects of Wiskostatin on the adhesiveness of HaCaT cells in an electric wounding assay. Probe-A2: medium control; probe-A3: Wiskostatin 100nM; probe-A6: Wiskostatin 10nM; probe-A7: Wiskostatin 1 nM. Shown are 400 (top), 4000 (middle) and 40,000 Hz (bottom);
Figure-5. Shows effects of Wiskostatin on HGF-induced adhesion of HaCaT cells in an electric wounding assay. All wells included HGF at 40ng/ml except where it is indicated. Probe-B2: medium control; probe-B3: Wiskostatin 100nM; probe-B6: Wiskostatin. 10nM; probe-B7: Wiskostatin 1 nM. Shown are 400 (top), 4000 (middle) and 40,000 Hz (bottom). Probe-A2: no HGF control;
Figure-6. Shows effects of Wiskostatin on the migration of HaCaT cells in an electric wounding assay. Probe-A2: medium control; probe-A3: Wiskostatin 100nM; probe-A6: Wiskostatin 10nM; probe-A7: Wiskostatin 1 nM. Shown are 400 (A-top), 4000 (B-middle) and 40,000 Hz (C-bottom);
Figure-7. Shows effects of Wiskostatin on HGF-induced the migration of HaCaT cells in an electric wounding assay. Probe-B2: medium control; probe-B3: Wiskostatin 100uM; probe-B6: Wiskostatin 10uM; probe-B7: Wiskostatin 1uM. Shown are 400 H. All wells except indicated were with HGF 40ng/ml;
Figure-8. Shows effects of an nWASP inhibitor, 187-1, on the migration and micromotion of HaCaT cells. Wild type HaCaT cells were treated with either control medium (A2), 187-1 at 10µM (A3), 178-1 at 1.0µM (A6), or 187-1 at 0.1µM (A7). The migration was recorded using ECIS9600 over a period up to 3 hours. The inhibitor has substantially increased the speed of migration/healing with the most obvious effect seen at 10µM. (graph generated from Experiment ID 20 Feb 2009 HaCaT 178-1.tc). A-shows traces of cell migration; B-shows the migration as calculated by Rb cell modelling (* p=0.0114 vs control); C: Concentration dependent stimulation of migration by 178-1; D-shows the modelling of micromotion, 8 p<0.05 vs control;
Figure-9. Shows evaluation of the role of nWASP in the migration of HaCaT keratinocytes. Top Left: A1 is the wild type HaCaT; A2 is HaCaT cells transfected with anti-nWASP transgene-2 and A3 with ant-nWASP transgene-3. A4 is cell free electrode control. Loss of nWASP from the cells (A2 and A3, also refer to figure-2) resulted in dramatic increase in cell migration (A2 and A3 vs A1). Top Right: all wells included HGF at 40ng/ml. A5 is the wild type HaCaT; A6 is HaCaT cells transfected with anti-nWASP transgene-2 and A7 with ant-nWASP transgene-3. A8 is cell free electrode control; Bottom two graphs: the effect of nWASP inhibitors on the growth of HaCaT cells (bottom left-Wiskostatin, Bottom right-187-1): Wiskostatin had limited inhibitory effect on the growth and only at a high concentration. 187-1 had not significant effect on the growth of the cells;
Figure-10. Shows nWASP had a profound impact on the function of endothelial cells. A: Cellular migration after nWASP expression. Electrode-A1: HECV wild type cells; A2: HECV/pcDNA-GFP plasmid control cells; A3/A4: duplicated assay for HECV transfected with pcDNA/GFP-nWASP7 plasmid. Forced expression of nWASP markedly reduced the migration of the cells. B: Quantified changes as shown by resistance in OHM. There was a significant reduction in the migration speed as shown by reduced resistance in nWASP over-expressing cells (p<0.05 vs wild type and control cells);
Figure-11. Shows A: Tubule formation 24 hours (X4 magnification). A1-A4-HECV-wt, B1-B4-HDCV-GFP control, C1-C4: HECV/nWASP7exp cells. 1-control; 2-cell treated with 187-1 (10uM, except C2A=1uM, and C2B-10uM); 3-cells treated with Wiskostatin shown are 100nM; 4-positive control (cells treated with HGF at 50ng/ml). Arrows indicate microtubules. Shown are micrographs 24 hours after seeding. B: The same experimental setting but photographed after 48 hours at X10 magnification;
Figure-12 Shows the effects of blocking nWASP by nWASP inhibitor 187-1 on the expansion of chronic wound tissue (WD). 187-1 treated in the expansion in areas of the chronic tissues compared with the control tissues during the 24 hour period;
Figure 13 shows the amino acid and cDNA sequence structure of nWASP and WASP;
Figure 14 shows nWASP expession was signficantly higher in wound tissues that fail to heal (chronic non-healing, n=51) than in wound that heal within 3 months (chronic healed, n=20);
Figure 15 shows the effect of systemic administration of 187-1 on the change of the size of wounds, shown are the percentage change of wound areas over day-1 in the respective groups;
Figure 16 shows the effect of systemic administration of 187-1 on the size of wounds, shown are size of the wounds in pixels (mean±SD);
Figure 17 shows topical application of 187-1 by Gel-B reduced the size of wounds, shown is the percentage change of wound areas over day-1 in the respective groups;
Figure-17b shows topical application of 187-1 by Gel-B reduced the size of wounds, shown are size of the wounds in pixels (mean±SD);
Figure 18 shows topical delivery of 187-1 by Gel A, shown is the percentage change of wound areas over day-1 in the respective groups;
Figure 18b shows topical application of 187-1 by Gel-A reduced the size of wounds, shown are size of the wounds in pixels (mean±SD);
Figure 19 shows the effect of systemic administration of Wiskostatin on wound healing, at both concentrations, there was a significant reduction of the size of wound by Wiskostatin, shown is the percentage change of wound areas over day-1 in the respective groups;
Figure 19b shows the effect of systemic administration of Wiskostatin on wound healing, at both concentrations, there was a significant reduction of the size of wound by Wiskostatin, shown are size of the wounds in pixels (mean±SD).
Figure 20 shows topical application of Wiskostatin on wound healing, shown are the percentage change of wound areas over day-1 in the respective groups;
Figure 20b shows topical application of Wiskostatin on wound healing, shown are the percentage change of wound areas over day-1 in the respective groups, shown are size of the wounds in pixels (mean±SD);
Figure 21 shows topical application of Wiskostatin using Gel A, shown is the percentage change of wound areas over day-1 in the respective groups;
Figure 21b shows topical application of Wiskostatin using Gel A, shown is the percentage change of wound areas over day-1 in the respective groups.

### MATERIALS AND PROCEDURE

1. Cells and Human keratinocytes and melanoma Cells (HaCaT-from The German Cancer Institute/Cell Service, Germany and A431 -from ATCC), human vascular endothelial cell, HECV (from Interlab, Italy) were used. Recombinant human HGF was from the research laboratory. nWASP inhibitors, Wiskostatin and 187-1 were from Merck Pharmaceuticals and TOCRIS, respectively. Tissue collection and preparation of RNA/cDNA bank from human wound/skin tissues. Fresh tissues from abnormal/chronic wounds (n=14), acute wounds (n=10) and normal skin from healthy volunteers (n=10) were collected under an approval from the local ethical committee(Ethical approval ID: 05/WSE03/92) and stored in -80°C until use. Written informed consent was obtained from each patient who agreed for a biopsy to be taken. Tissues were frozen sectioned on a cryostat (Leica). A portion of the sections were kept for histological analysis. Approximate 20 sections were pooled and homogenised using a hand-held homogenizer using a procedure to extract RNA from the tissues. RNA extracted from the tissues was quantified and cDNA was subsequently generated using a RT kit. Abnormal/chronic *wound tissues were* from patients with abnormal/chronic leg ulcers. *Acute wound tissues were* obtained from patients with acute surgical wounds after undergoing excision of pilonidal disease. *Normal tissues* were from normal volunteer's normal skin.
2. Analysis of nWASP gene transcripts was carried out using conventional and quantitative real time PCR (Icycler IQ, Bio-Rad) on human wound and skin tissues and cells. For conventional PCR, primers used were: NWASPF8 (5'agtccctcttcactttcctc'3) and NWASPR8 (5'gcttttcccttcttcttttc'3) and NWASPF9 (5'attttcatacctttgctgga'3) and NWASPR9 (5'taacagcttcaacacctcct'3). For Q-PCR primers used were nWASPF1 and NWASPZR1 (5'gagctggatgagaacaacac'3) and (5'actgaacctgaccgtacaaaagaagtggcaggaagagt'3).
3. The study adopted the Ampliflor quantitation technology, in which one set of gene specific primers and a Uniprimer probes were used, in combination with quantitative PCR master mix. The reaction was carried out using ICycler^{IQ} (Bio-Rad). An internal standard was employed for quantitation purpose. In all the assays, GAPDH and actin were amplified and used as the house keeping controls. Levels of a specific gene was normalised to the expression level of GAPDH in the respective sample for example, nWASP, GAPDH and actin transcripts (mRNA) were measure in the same sample. nWASP was then normalised to GAPDH.
4. Evaluation of cells response to wound stress after n-WASP is targeted. Here, an in vitro cell model, based on the ECIS system, was adopted. Briefly, cells were allowed to sit in the electric arrays and an 8E10 format was used. Cells in fixed number and fixed volume of culture medium were added into each of the respective wells, which had either control medium or a specific inhibitor to N-WASP, namely Wiskostatin or 187-1. In selected wells, a cell migration inducer, HGF was also included in the absence or presence of the nWASP inhibitors. Cells were investigated for the following functions: the adhesiveness to the matrix surface and the migration capacity. The first was recorded immediately after plating of the cells into each well at 3 frequencies: 400Hz, 4,000Hz, 40,000 Hz. The frequencies were chosen following a frequency scanning (Figure-3). Two main cell functions were thus tested here: the adhesiveness of the cells to matrix and cell migration.
5. Creation of cell models. We have created a new cell model, based on a human keratinocyte cell line, HaCaT, which expressed high levels of n-WASP. By using anti-nWASP transgenes, we successfully knocked out the expression of n-WASP from the cells and subsequently used the cells in our testing. Briefly, the secondary structure (Figure-2) of human nWASP was generated using Zuker's RNA mFold software. Three suitable sites for targeting within the nWASP mRNA were identified. These sites fulfil the following criteria: GTC/ATC/TTC sequence in the mRNA sequence and GTC/ATC sequence situated in a loop (a large loop preferred) region and not in a stem region. Touch-down PCR was used to generate PCR-based ribozyme/RNA using paired oligos. Pstl and Spel restriction sites were introduced during the PCR reaction. Three anti-nWASP transgenes were prepared using the following short oligos: nWASPRib1F (5'Ctgcaggagttctttgaccacatacagttccctgatgagtccgtgagga'3) and nWASPRib1R (5'Actagttggtgcagttatatgcagcag**A**tttcgtcctcacggact'3), nWASPrib2F (5'Ctgcagacaagcaacaccactgcacttctttctgatgagtccgtgagga'3) and nWASPRib2R (5'Actagttatatgcagcag**ATC**ggaactgtatgtg**G**tttcgtcctcacggact'3), nWASPRib3F (5'Ctgcaggtgcagctgtgggagctcttctgatgagtccgtgagga'3) and nWASPRib3R (5'Actagtgctaggggaagaggcgctcctcccccaccacc**T**tttcgtcctcacggact'3). PCR products generated antisense-hammerhead ribozyme, flanked by pstl and Spel restriction sites, were T-A cloned into a pEF6/V5/His TOPO cloning vector which used EF6 promoter for mammalian expression (Invitrogen, Paisley, Scotland, UK), which was amplified in the OneShot^{™} E.coli (Invitrogen). Clones with correct oriented insert were verified using PCR. Plasmid was subsequently purified from the bacterial preparation and used for transfection of HaCaT cells by way of electroporation (EasyJet, Flowgen, England, UK). Following selection, transgenes-1, 2 and 3 were found to be active in knocking down nWASP mRNA from HaCaT cells.
6. For cell adhesion assay, we adopted the ECIS methods by employing ECIS9600 and 1600R models of these instruments. Cells were plated into the corresponding wells in a 8W10E array, in which different concentration of Wiskostatin was included (please see Figure legends). The adhesion was recorded at 400Hz, 4,000Hz and 40,000 Hz over a 3 hour period.
7. Cell migration assay. This was essentially the same as the adhesion assay, except that cells were allowed to reach confluence first. They were then electrically wounded at 5 volts for 60 seconds. Electrical sensing was immediately applied after wounding for over a period up to 15 hours, at varying frequencies. Furthermore, we also determined the micromotion of cells under the same experimental setting. This is one further type of function that our instrument measures, in that quiescent cells (no wounding) were evaluated by the instrument over a period of minutes at a 0.1 second interval. The instrument would predict the micromotion between cell membrane and the electrodes. Micromotion reflects the subtle interaction between cell membrane and electrode and partly reflects the migration capacity of the cells. Micromotion was recorded at 15 minute intervals and analysed using cell modelling.
8. *Ex vivo* effects of nWASP inhibitors in chronic human wound tissues. This was based on an *ex vivo* model that we previously established (Jiang and Harding 1998). Briefly, fresh biopsies from abnormal/chronic wounds were immediately placed in a purposely made buffer that mimic the physiological fluid and with a mixture of antibiotics. The tissues were finely minced using sterile scalpel to sizes below 1 mm in diameter. After extensive washing in the buffer, the living tissues were immediately embedded in extracellular matrix gel as we previously described in Jiang WG and Harding KG. Enhancement of expansion of wound tissue and angiogenesis by matrix embedded fibroblasts (Dermagraft), a role for hepatocyte growth factor/scatter factor. International Journal of Molecular Medicine, 1998, 2 (2), 203-210. The gels and the topping solution include test materials (cytokines and nWASP inhibitors). The tissues were photographed daily. The degree of tissue expanded from the implanted tissues was calculated using the imager as we previously reported in Jiang WG and Harding KG. Enhancement of expansion of wound tissue and angiogenesis by matrix embedded fibroblasts (Dermagraft), a role for hepatocyte growth factor/scatter factor. International Journal of Molecular Medicine, 1998, 2 (2), 203-210.
9. nWASP and angiogenesis. We used an in vitro angiogenesis model, endothelial tubule forming assay. Briefly, endothelial cells with differential expression of nWASP were sandwiched between layers of matrix proteins and allowed to form microtubules. The cells were also treated with known angiogenic factors or nWASP inhibitors. The tubules were evaluated using a time lapse video recorder and image analysis tools. The endothelial cells used in this study, HECV, which were negative in nWASP expression, were transfected with a mammalian expression plasmid that carried full length human nWASP coding region. This was prepared from normal breast tissue cDNA (using primer sets: 5'atgagctccgtccagcag'3 and 5'tcagtcttcccactcatcatc'3,) was T-A cloned into an pcDNA-NT GFP-TOPO (Invitrogen) plasmid, selection marker G418). The impact of nWASP expression and nWASP inhibitors on tubule formation was evaluated. Furthermore, cell functions including cell attachment and cell migration after forced expression of nWASP in HECV cells were also determined using the ECIS method.
10. Statistical analysis was conducted using Minitab, SPSS and an online Chisquare service tool (http://www.people.ku.edu/~preacher/chisq/chisq.htm).

### In Vivo Studies

### In vivo tolerance test.

First, the main tolerance tests were conducted using the CD-1 athymic mice (Charles River Laboratories), owing to their slow and steady rate of growth and easier to observe changes in the skin (hairless) and other possible side effects. Briefly, CD-1 of 4-6 weeks old, 20g in weight, were housed in filter topped cages. 187-1 (MW 1784, dissolved in BSS buffer) and Wiskostatin (MW 426, dissolved in DMSO and diluted in BSS), were injected, via the intraperitoneal route, on a daily basis. Both compounds were given at 1 and 10M final concentration in 100ul in volume. Dosages administered were 1 and 10uM for each compounds, equivalent to 1.8g/kg/day and 17.8g/kg/day for 187-1 and 0.43g/kg/day and 4.3g/kg/day. CD-1 were observed daily, weighed twice weekly. An additional tolerance and efficacy test was carried out using the db/db strain.

### In vivo efficacy test and wound healing.

The diabetic strain of db/db was obtained from Harlan. 4-6 weeks old with body weight at 20g were used. Creation of a wound was according to a recently described method (Cho et al 2006). Briefly, after being housed for a week, the db/db mice were first ear-pieced using an ear puncher, in order to create a wound (hole) of 1mm in diameter. The following day after wound creation, all the db/db were weighed, wound photographed using a digital camera. Treatment was given systemically (by IP injection) or topically (by manually applying the compounds in gel into the wound area). Both treatments were given every other day. Images were obtained weekly. The size of the wounds was determined using an image analysis software. Data are given in two ways:
1. the area of the wounds in pixels. Two sample student t test was used for statistical analysis.
2. change of the size of wound over the starting point calculated using: (area at a given point - area at the starting point)/(area of the starting point)X100. Bonferroni model was used for data analysis.

### Formulation of the compounds.

*For systemic application,* 187-1 was dissolved in BSS and diluted in the same for the required concentration. Wiskostatin was first dissolved in DMSO at concentration of 5mg/ml. The DMSO solution was then gradually diluted in BSS in order to avoid precipitation. The solutions were prepared such that each 100ul contained the correct amount of compounds and was aliquatted and stored at - 20°C until used. The compounds were injected every other day by the IP route. Dosages administered were 0.5 and 5uM or 0.89g/kg/day and 8.9g/kg/day for 187-1 and 1 and 10uM or 0.43g/kg/day and 4.3g/kg/day. Treatment was given every other working day (Monday, Wednesday and Friday) and the images obtained on Wednesday.

*For tropical application*, we used two conventional carrier gels (purchased from Pharmacy of University Hospital of Wales) that are currently used in wound care. From the concentrated master stock of 187-1 and Wiskostatin, 100ul of the stock solution was mixed with 2 grams of the respective gels (equivalent to 1 mg of the respective compound for 1 gram of the respective gel), followed by low speed homogenisation using a hand held homogeniser, for 2 minutes. The newly formulated gels which showed no sign of changes of the strength and consistency, were stored at 4°C until used. For use, a small amount (150ul) of the gel was applied to the wound area and gently rubbed in using fingers. Treatment was given every other working day (Monday, Wednesday and Friday) and the images obtained on Wednesday.

### RESULTS

### N-WASP is differentially expressed in normal skin, acute and abnormal/chronic wound tissues

By examining the levels of nWASP mRNA transcripts in human skin and wound tissues, it was revealed that abnormal/chronic wound tissues had significantly higher levels of the nWASP transcripts than normal skin and acute tissues (Figure-1).

### N-WASP inhibitor, Wiskostatin, increase the capability of cell adhesion to matrix

HaCaT cell expressed nWASP as shown by conventional RT-PCR (Figure-2-Right). In Figure-2 right, there is a faint band indicating the presence of nWASP transcript in wild type, control and in transgene-3 cells, indicating that transgene-3 was not very active.

As shown in Figure-4, inclusion of Wiskostatin increased the adhesiveness of HaCaT cells to matrix surface, particularly when recorded at 40,000 Hz, at which minor differences between cells are visible (please see Figure-3).

As shown in Figure-5, where cells were treated with HGF, a cytokine that increases the motility of cell, HGF increased the adhesiveness of the cells compared with control cells. Inclusion of Wiskostatin further increased the adhesiveness, as shown by a rapid return to normal level after the cells were wounded.

### Wiskostatin increased the migration of HaCat cells

Using a similar assay, we further evaluated the impact of inhibition of N-WASP on the migration of HaCaT cells.

Figures-6 and -7 shows the effects of Wiskostatin on the migration of HaCat cells. For example, measured at 400Hz (figure-6A), inclusion of Wiskostatin at 1 nM and 10nM resulted in a rapid rise of resistance, following wounding.

### 187-1, an nWASP inhibitor displayed a similar stimulatory effect on the healing of the keratinocyte monolayer

Using the above methods, we tested the effect of 187-1, a small nWASP inhibitor (TOCRIS, MW 1784), on the migration of HaCaT cells. As shown in Figure-8a, over a concentration range tested, 187-1 showed a stimulatory effect on the migration of the cells with the strongest effect seen at 10µM. The statistical difference was demonstrated using the Rb Cell Modelling methods in which cells with 10µM 187-1 showed a rapid increase in migration speed (p=0.0114) (Figure-8B). Furthermore, a concentration dependent effect was seen with the effect reaching maximum after 10µM (Figure-8C).

Micromotion analysis has revealed that cells given 187-1 also had a significant increase in micromotion (Figure-8D).

Finally, all the effects of Wiskostatin and 187-1 on the aforementioned cell functions were achieved at concentrations that are far below those that may cause toxicity and growth changes (figure-9C bottom).

### Knocking down n-WASP from keratinocytes drastically increased the

### migration of the cells

Based on the secondary structure of nWASP mRNA (Figure-2), three sites were found suitable for targeting. Three anti-nWASP transgenes were created. In transfecting the HaCaT cells, transgenes-2 and -3 were highly active (Figure-2) and the cells that carried these two transgenes were subsequently used for testing.

As shown in Figure-9 (top left), cells that carried anti-nWASP transgenes and so lost nWASP mRNA had dramatically increased rate of healing/migration when compared with the control cell. Using HGF as a migration inducing agent, it was found that HGF increased the migration of control HaCaT cells and also has some limited effect on the nWASP transgene carrying cells (Figure-9 top right)

### Impact of nWASP over-expression on in vitro angiogenesis and migration of vascular endothelial cells

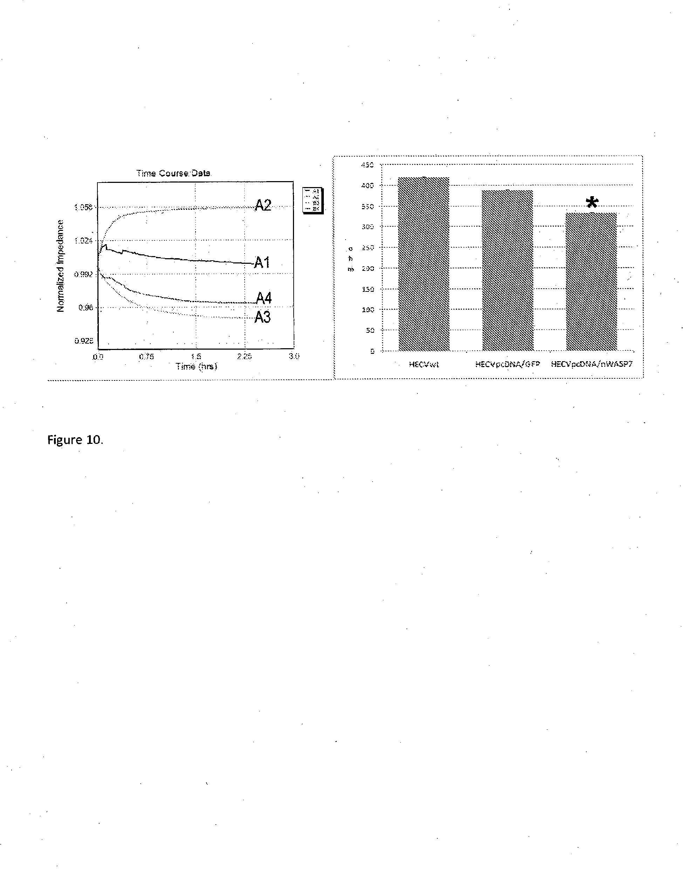

When HECV cells were forced to express nWASP, there was a significant reduction in the migration as shown in Figures 10A and 10b.

Using in vitro tubule formation assays, it was found that neither 187-1 nor Wiskostatin had a major impact on the tubule formation from HECV cells, a cell line that is negative for nWASP expression. However, as shown in Figure-11 A, when HECV cells were transfected and forced to express nWASP, their responses to the inhibitors changed substantially, particularly with 187-1, in that the presence of 187-1 markedly increased the formation of microtubules in nWASP expression HECV cells. Figure-11 B shows tubule forming after 48 hours. Both nWASP and 187-1 now show a strong stimulatory effect on tubule forming, in HECV cells that were forced to express nWASP.

### nWASP inhibitors and expansion of chronic wound tissues

We tested the impact of nWASP inhibitors on the expansion of wound tissues using an ex vivo model that we established. As shown in Figure-12, inclusion of 187-1 in the 3-D system resulted in expansion of the chronic tissues, whereas no expansion was seen in control conditions over the same period.

### Validation study on chronic wound tissues revealed a significant over-expression of nWASP in chronic non-healing wound tissues

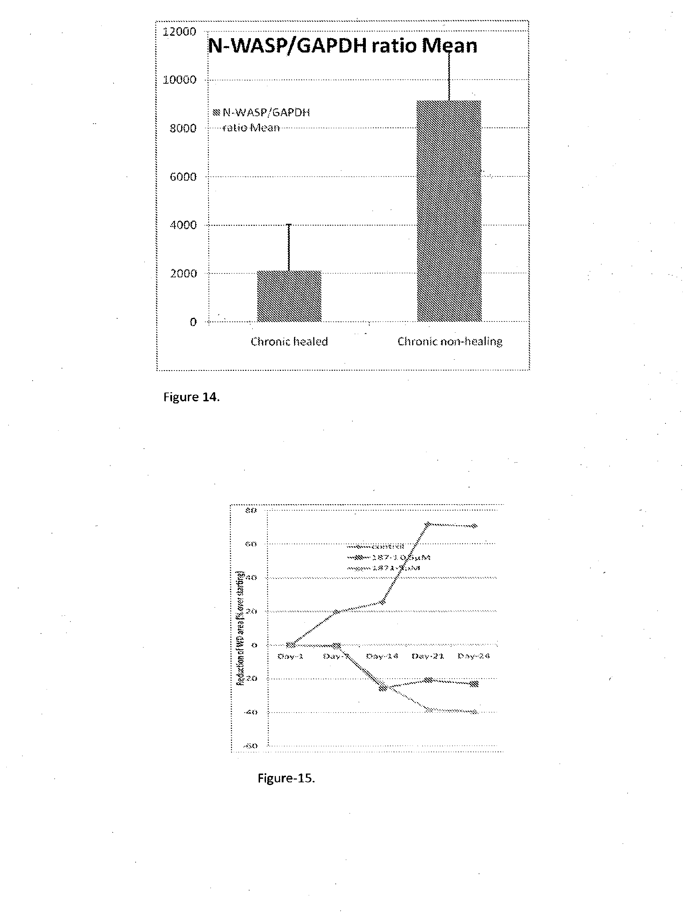

Using an independent cohort of chronic tissues, we have shown wounds that failed to heal had a significantly higher levels of nWASP that which had healed (p<0.05) (figure 14).

### 187-1 and wiskostatin are well tolerated

We have delivered the compounds systemically on a daily basis in our tolerant test, for a two week period in athymic CD-1. Throughout the study, we did not observe any side effects. There was no weight loss and no signs of any changes in the skin in any of the groups. The compounds were also well tolerated in the db/db strain, in that administration on alternative date rendered no side effects.

### 187-1, administered systemically, accelerated wound healing without producing any side effects

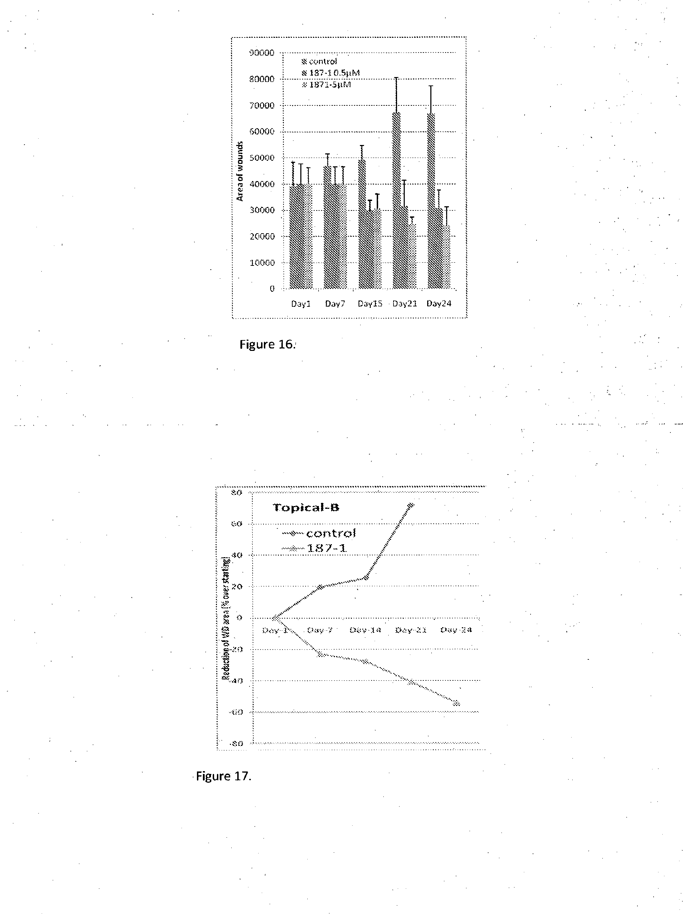

Two concentrations of 187-1 were given systemically, 0.5 and 5 µM (equivalent to 0.89g/kg/day and 8.9g/kg/day). After two weeks, wounds in the treated group were significantly smaller than the control group as shown in Figure 15 (*p=0.037 and p=0.04, control vs 187-1, 0.5 and 5µM resectively, 2-Way ANOVA with Bonferroni model)*. Figure 16 showed the change of wound area.

### 187-1, administered topically, accelerated wound healing without producing any side effects

Topical application of 187-1 in both gels A and B showed a significant effect after three weeks, *(p=0.028 and p=0.045, control vs 187-1, in topical-B and Topical-A applications respectively, 2-Way ANOVA with Bonferroni model)* (figure-17 and 17b, 18 and 18b).

### Wiskostatin, systemically delivered, significantly accelerated wound healing without producing any side effects

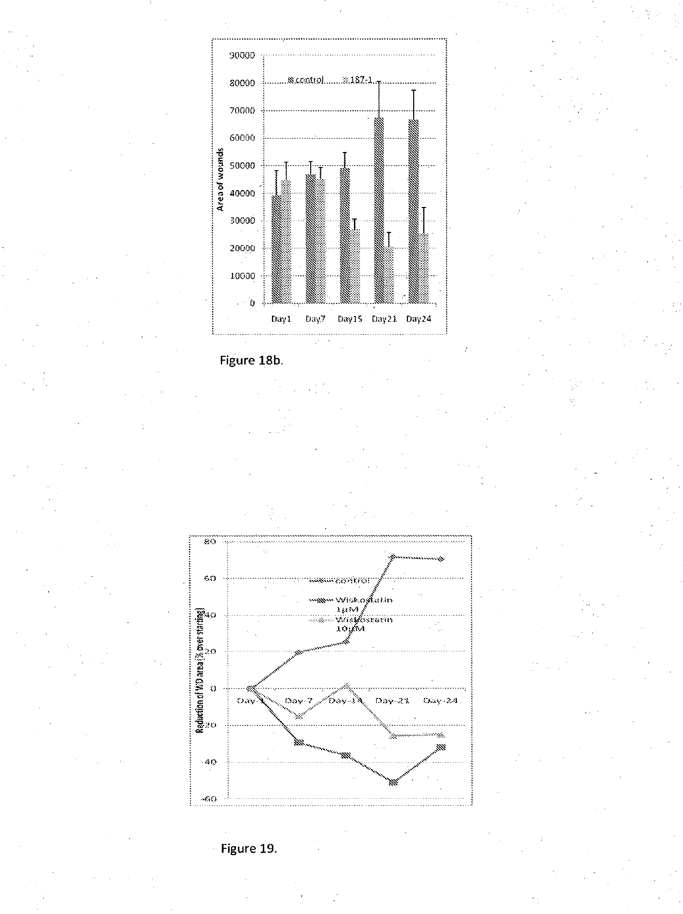

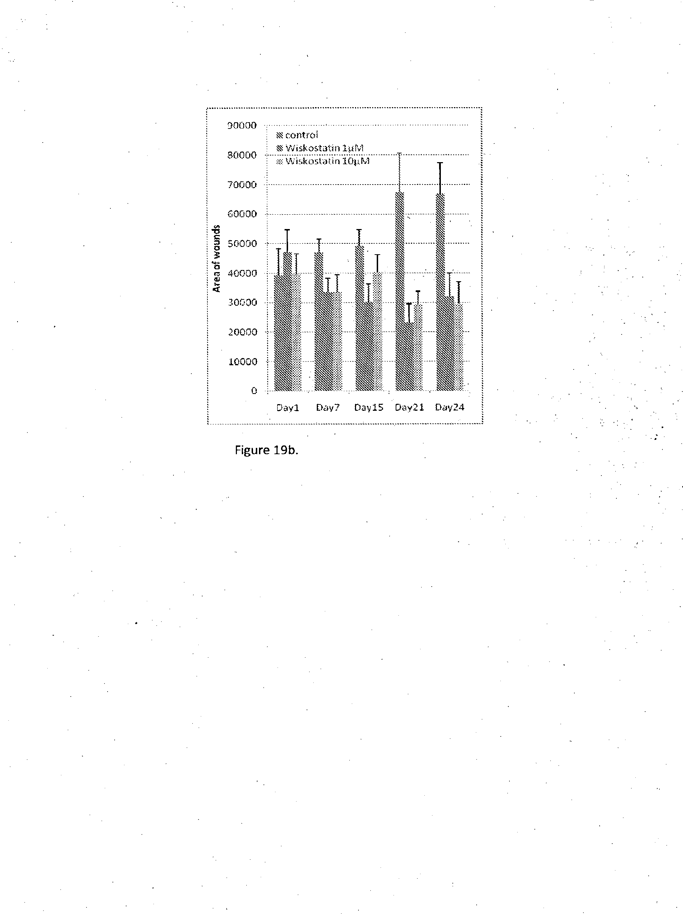

At 1 and 10µM, Wiskostatin had significantly reduced the size of the wounds in comparison with control (p=0.017 and p=0.04, control vs Wiskostatin, 1 and 10µM respectively, 2-Way ANOVA with Bonferroni model) after three weeks (figure 19 and 19b).

### Topical application of Wiskostatin, also showed a significant effect after three weeks

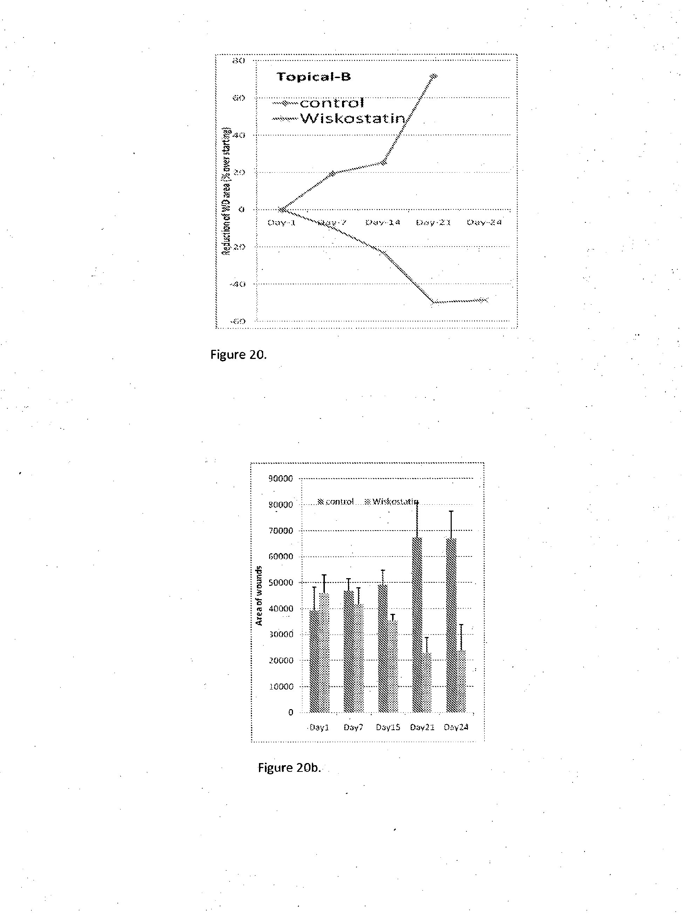

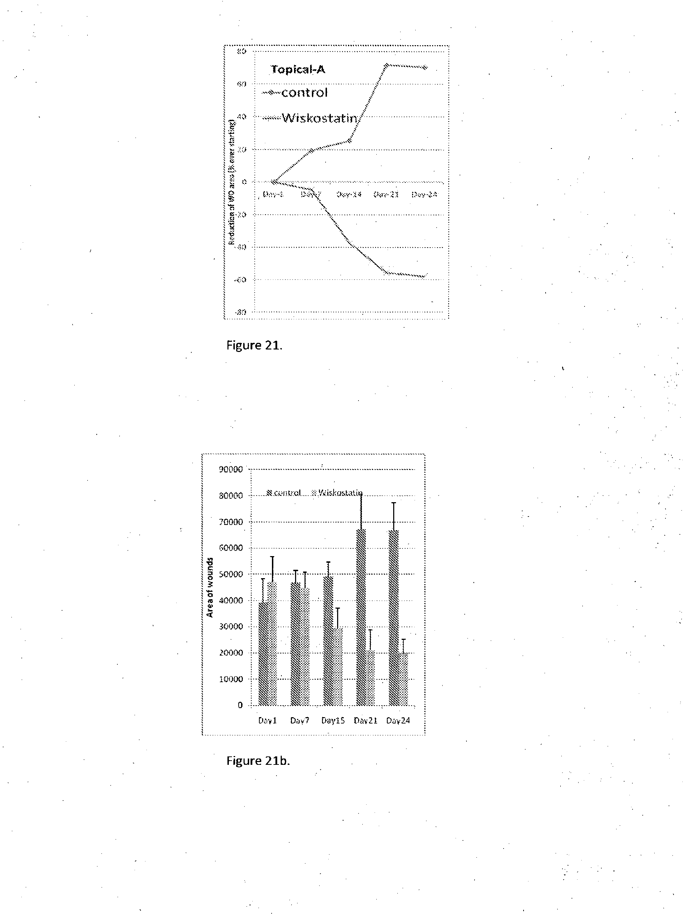

Using either carrier gel Wiskostatin showed a significant effect (p=0.044 and p=0.044, control vs 187-1, in topical-B and Topical-A applications respectively, 2-Way ANOVA with Bonferroni model) (figure 20 and 20b, 21 and 21b).

### Summary

The main findings of the present study can be summarised in the following:
Abnormal/chronic human wounds have increased levels of nWASP as demonstrated by quantitative transcript analysis; and using an exogenous nWASP inhibitor, such as Wiskostatin, can increase the migration and healing speed of wounded tissue or using knock down nWASP transcripts can also increase the migration and healing speed of wounded tissue.

These findings collectively show that nWASP is critical in controlling the migration and healing of wounds. Together with the increased expression of nWASP in abnormal/chronic wounds, it is plausible to suggest that high levels of nWASP in a given wound may indicate that nWASP hampers the healing process of the wound, which may primarily be due to reduced ability of cells type, to migrate into the wound. Thus, both in vitro and clinical data point to nWASP being an important therapeutic target in abnormal/chronic wounds.

## Claims

1. A therapeutic comprising an inhibitor of either, or both, nWASP gene expression or nWASP protein activity; or an inhibitor of a protein that is at least 90% homologous to nWASP and that modulates actin assembly, for use in the treatment of mammalian chronic wounds.

2. A therapeutic for use according to claim 1 wherein said inhibitor is an inhibitor of nWASP protein function.

3. A therapeutic for use according to claim 2 wherein said inhibitor is selected from the group consisting of: a nWASP binding agent that binds, either reversibly or irreversibly, to inhibit protein function such as an antibody or a known, or synthesized, nWASP antagonist; or an agent that works upstream or downstream of the nWASP signalling mechanism to inhibit nWASP function.

4. A therapeutic for use according to claim 3 wherein said inhibitor is Wiskostatin (Merck Pharmaceuticals) or 187-1 (TOCRIS).

5. A therapeutic for use according to claim 1 wherein said inhibitor is an inhibitor of nWASP gene expression.

6. A therapeutic for use according to claim 5 wherein said inhibitor is selected from the group consisting of: anti-sense DNA or RNA, siRNA, or ribozymes, either naked or in the form of plasmid or viral vectors.

7. A therapeutic for use according to claim 6 wherein said inhibitor is anti-nWASP ribozyme/RNA transgene selected from the group comprising:
a) transgene 1
b) or transgene 2
c) or transgene 3

8. A therapeutic for use according to any preceding claim wherein the therapeutic is formulated for use in treating human wounds.

9. A therapeutic for use according to any preceding claim wherein the therapeutic is formulated for topical application.

10. A therapeutic for use according to any preceding claim wherein the therapeutic is formulated for application to a dressing or impregnation in a dressing.

11. A pharmaceutical composition for use in the treatment of mammalian chronic wounds comprising a therapeutic according to any preceding claim together with a pharmaceutically acceptable carrier.

12. A kit for use in the treatment of mammalian chronic wounds wherein said kit comprises:
a) at least one therapeutic according to claims 1-10 or a composition according to claim 11; and
b) at least one dressing for applying to said wound.

13. A combination therapeutic for use in the treatment of mammalian chronic wounds according to claim 1 comprising an inhibitor of:
a) n-WASP gene expression according to any one of claims 1-4; and
b) an inhibitor of n-WASP protein activity according to any one of claims 5-7

## Patentansprüche

1. Therapeutikum, umfassend einen Inhibitor von einem oder beiden, nWASP-Genexpression oder nWASP-Proteinaktivität; oder einen Inhibitor eines Proteins, das mindestens 50 % homolog zu nWASP ist und das Aktinzusammensetzung moduliert, zur Verwendung bei der Behandlung von chronischen Wunden von Säugern.

2. Therapeutikum zur Verwendung nach Anspruch 1, wobei der Inhibitor ein Inhibitor von nWASP-Proteinfunktion ist.

3. Therapeutikum zur Verwendung nach Anspruch 2, wobei der Inhibitor ausgewählt ist aus der Gruppe bestehend aus: einem nWASP bindenden Mittel, das bindet, um die Proteinfunktion entweder reversibel oder irreversibel zu hemmen wie ein Antikörper oder ein bekannter oder synthetisierter nWASP-Antagonist; oder ein Mittel, das dem nWASP-Signalisierungsmechanismus vorgeschaltet oder nachgeschaltet wirkt, um die nWASP-Funktion zu hemmen.

4. Therapeutikum zur Verwendung nach Anspruch 3, wobei der Inhibitor Wiskostatin (Merck Pharmaceuticals) oder 187-1 (TOCRIS) ist.

5. Therapeutikum zur Verwendung nach Anspruch 1, wobei der Inhibitor von WASP Genexpression ist.

6. Therapeutikum zur Verwendung nach Anspruch 5, wobei der Inhibitor ausgewählt ist aus der Gruppe bestehend aus: Antisense-DNA oder RNA, siRNA oder Ribozymen, entweder nackt oder in Form von Plasmid und viralen Vektoren.

7. Therapeutikum zur Verwendung nach Anspruch 6, wobei der Inhibitor ein Anti-nWASP-Ribozym/RNA-Transgen ist ausgewählt aus der Gruppe bestehend aus:
a) Transgen 1
b) oder Transgen 2
c) oder Transgen 3

8. Therapeutikum zur Verwendung nach einem der vorhergehenden Ansprüche, wobei das Therapeutikum zur Verwendung bei der Behandlung von menschlichen Wunden formuliert ist.

9. Therapeutikum zur Verwendung nach einem vorhergehenden Anspruch, wobei das Therapeutikum zur topischen Anwendung formuliert ist.

10. Therapeutikum zur Verwendung nach einem vorhergehenden Anspruch, wobei das Therapeutikum zum Auftrag auf einen Verband oder Imprägnierung in einem Verband formuliert ist.

11. Pharmazeutische Zusammensetzung zur Verwendung bei der Behandlung chronischer Wunden von Säugern, die ein Therapeutikum nach einem vorhergehenden Anspruch zusammen mit einem pharmazeutisch annehmbaren Träger umfasst.

12. Kit zur Verwendung bei der Behandlung chronischer Wunden von Säugern, wobei das Kit umfasst:
(a) mindestens ein Therapeutikum nach Ansprüchen 1-10 oder eine Zusammensetzung nach Anspruch 11; und
(b) mindestens einen Verband zum Auftragen auf die Wunde.

13. Kombinationstherapeutikum zur Verwendung bei der Behandlung chronischer Wunden von Säugern nach Anspruch 1, umfassend einen Inhibitor von:
a) n-WASP-Genexpression nach einem der Ansprüche 1-4; und
b) einen Inhibitor von n-WASP Proteinaktivität nach einem der Ansprüche 5-7.

## Revendications

1. Produit thérapeutique comprenant un inhibiteur soit de l'expression de gène nWASP soit de l'activité de la protéine nWASP, soit des deux fonctions ; ou un inhibiteur d'une protéine qui est homologue au moins à 90 % à nWASP et qui module l'assemblage de l'actine, pour une utilisation dans le traitement des plaies chroniques de mammifères.

2. Produit thérapeutique pour une utilisation selon la revendication 1, dans lequel ledit inhibiteur est un inhibiteur de la fonction de protéine nWASP.

3. Produit thérapeutique pour une utilisation selon la revendication 2, dans lequel ledit inhibiteur est choisi dans le groupe consistant en : un agent de liaison nWASP qui se lie, soit de façon réversible soit de façon irréversible, pour inhiber la fonction de protéine tel qu'un anticorps ou un antagoniste nWASP, connu ou synthétisé ; ou un agent qui intervient en amont ou en aval du mécanisme de signalisation nWASP pour inhiber la fonction nWASP.

4. Produit thérapeutique pour une utilisation selon la revendication 3, dans lequel ledit inhibiteur est la Wiskostatine (Merck Pharmaceuticals) ou le produit 187-1 (TOCRIS).

5. Produit thérapeutique pour une utilisation selon la revendication 1, dans lequel ledit inhibiteur est un inhibiteur de l'expression de gène nWASP.

6. Produit thérapeutique pour une utilisation selon la revendication 5, dans lequel ledit inhibiteur est choisi dans le groupe consistant en : ADN ou ARN anti-sens, petit ARN interférent ou ribozymes, soit nus soit sous forme de vecteurs plasmidiques ou viraux.

7. Produit thérapeutique pour une utilisation selon la revendication 6, dans lequel ledit inhibiteur est un transgène d'ARN/ribozyme anti-nWASP choisi dans le groupe comprenant :
a) le transgène 1
b) ou le transgène 2
c) ou le transgène 3

8. Produit thérapeutique pour une utilisation selon l'une quelconque des revendications précédentes, dans lequel le produit thérapeutique est formulé pour une utilisation dans le traitement des plaies humaines.

9. Produit thérapeutique pour une utilisation selon l'une quelconque des revendications précédentes, dans lequel le produit thérapeutique est formulé pour une application topique.

10. Produit thérapeutique pour une utilisation selon l'une quelconque des revendications précédentes, dans lequel le produit thérapeutique est formulé pour une application sur un pansement ou l'imprégnation d'un pansement.

11. Composition pharmaceutique pour une utilisation dans le traitement des plaies chroniques de mammifères comprenant un produit thérapeutique selon l'une quelconque des revendications précédentes associé à un support pharmaceutiquement acceptable.

12. Kit pour une utilisation dans le traitement des plaies chroniques de mammifères dans lequel ledit kit comprend :
a) au moins un produit thérapeutique selon les revendications 1 à 10 ou une composition selon la revendication 11 ; et
b) au moins un pansement à appliquer sur ladite plaie.

13. Association de produits thérapeutiques pour une utilisation dans le traitement des plaies chroniques de mammifères selon la revendication 1 comprenant un inhibiteur de :
a) l'expression de gène n-WASP selon l'une quelconque des revendications 1 à 4 ; et
b) un inhibiteur de l'activité protéine n-WASP selon l'une quelconque des revendications 5 à 7.
